# EUROPEAN PATENT APPLICATION

(11) **EP 4 706 639 A1**
(43) Date of publication of application: **11.03.2026**
(21) Application number: 24386104.4
(22) Date of filing: 06.09.2024
(51) Int. Cl.: A61K 9/48, A61K 38/00, A61L 27/34, A61P 29/00, C07K 14/315, C07K 14/47

(54) **COMPLEMENT BINDING FUSION PROTEIN**

(71) Applicant: Invizius Limited, Edinburgh, Lothian EH4 2HS (GB)
(72) Inventor: Herbert, Andrew Peter, Edinburgh EH4 2HS (GB); Barlow, Paul, Edinburgh EH4 2HS (GB); Makou, Elisavet, Edinburgh EH4 2HS (GB)
(74) Representative: HGF

(57) **Abstract**

A fusion protein of human serum albumin to *Streptococcus pneumoniae* PspCN, which binds complement factor H, is provided. The fusion protein has a structure A-L-B, wherein: A comprises a sequence that has at least 90% similarity to SEQ ID NO :1 (amino acids 25-609 of human serum albumin, HSA); L comprises a linker or a chemical bond; B comprises a sequence that has at least 90% similarity to SEQ ID NO:2 (amino acids 69-148 of PspC from the TIGR4 strain of *Streptococcus pneumoniae*). Also provided are medical devices, especially for use in haemodialysis, at least partially coated with the fusion protein and uses of the fusion protein.

## Description

### Field

The present disclosure relates to fusion proteins, more specifically to fusion proteins that bind to complement factor H and uses thereof.

### Background

The complement system is a set of 40-50 proteins in the blood that provides a first line of defence against infection. An improperly regulated complement system can damage host or self-cells as well as bacterial ones. It can thus cause or exacerbate the symptoms of many diseases. There is consequently much interest in developing reagents that monitor or modulate the proteins of the complement system.

Human complement factor H (CFH) is a protein that acts selectively on self-surfaces in order to protect them from complement system-mediated damage. Yet many pathogens can exploit host CFH for their own purposes. Studying how CFH (consisting of 20 complement control protein (CCP) modules, also referred to as short consensus repeats (SCRs) or sushi domains) interacts with the relevant microbial proteins has provided clues as to how CFH can be captured and its activity modulated by polypeptides produced in the laboratory.

PspC is one of four designations for a pneumococcal surface protein whose gene is present in approximately 75% of all *Streptococcus pneumoniae* strains. Under the name SpsA, the protein has been shown to bind secretory immunoglobulin A (S. Hammerschmidt, S. R. Talay, P. Brandtzaeg, and G. S. Chhatwal, Mol. Microbiol. 25:1113-1124, 1997). Under the name CbpA, the protein has been shown to interact with human epithelial and endothelial cells (C. Rosenow et al., Mol. Microbiol. 25:819-829, 1997). The gene is paralogous to the PspA gene in *S*. *pneumoniae* and was thus called PspC (A. Brooks-Walter, R. C. Tart, D. E. Briles, and S. K. Hollingshead, Abstracts of the 97th General Meeting of the American Society for Microbiology 1997). Under the name HIC, the protein has been shown to interact with human factor H (Janulczyk R., lannelli F., Sjoholm A.G., Pozzi G., Bjorck L. J. Biol. Chem. 275:37257-37263,2000). A detailed description of PspC is provided in Brooks-Walter et al., "The pspC Gene of Streptococcus pneumoniae Encodes a Polymorphic Protein, PspC, Which Elicits Cross-Reactive Antibodies to PspA and Provides Immunity to Pneumococcal Bacteremia" Infect Immun. 1999 December; 67(12): 6533-6542. PMCID: PMC97064. In this paper sequence comparisons of five published and seven new alleles reveal that this gene has a mosaic structure, and modular domains have contributed to gene diversity during evolution. Two major clades exist: clade A alleles are larger and contain an extra module that is shared with many PspA alleles; clade B alleles are smaller and lack this PspA-like domain. All alleles have a proline-rich domain and a choline-binding repeat domain that show 0% divergence from similar domains in the PspA protein.

The present inventors produced a *Streptococcus pneumoniae* PspC truncation (PspCN) that binds CFH irreversibly (on the biological timescale) primarily *via* interactions with CCPs 8-10. PspCN-captured CFH adopts a conformation in which a chemical cross-linker can conjoin its CCP 5 and CCP 18, as well as its CCP 7 and CCP 20; it is of interest that CCPs 7 and 20 mediate self-surface recognition and are dual binding sites for numerous other CFH-interacting bacteria. This truncation is described in detail in International patent application WO 2015/055991 in the name of the University Court of the University of Edinburgh, which is incorporated herein by reference in its entirety.

However, there remains an unmet need for methods and compositions for selectively binding complement components, in particular CFH, to solid supports and for modulating complement activity. The ability to modulate complement activity opens up opportunities, *inter alia*, for treating diseases associated with aberrant complement activity.

### Summary

According to a first aspect there is provided a fusion protein having a structure A-L-B, wherein: A comprises a sequence that has at least 90% similarity to SEQ ID NO :1; L comprises a linker or a chemical bond; B comprises a sequence that has at least 90% similarity to SEQ ID NO:2.

It will be understood that the structure "A-L-B" corresponds to a fusion protein having a first subunit A and a second subunit B connected to one another by linker or chemical bond L.
SEQ ID NO:1 corresponds to amino acids 25-609 of human serum albumin and is provided below: 
SEQ ID NO:1






 




SEQ ID NO:2 corresponds to amino acids 69-148 of PspC from the TIGR4 strain of *Streptococcus pneumoniae* and is provided below:

SEQ ID NO:2
SERDKARKEVEEYVKKIVGESYAKSTKKRHTITVALVNELNNIKNEYLNKIVESTSESQL
QILMMESRSKVDEAVSKFEK

Subunit A may comprise a sequence that has at least 95% similarity to SEQ ID NO:1. Subunit A may comprise a sequence that has at least 99% similarity to SEQ ID NO:1.

Subunit B may comprise a sequence that has at least 95% similarity to SEQ ID NO:2. Subunit B may comprise a sequence that has at least 99% similarity to SEQ ID NO:2.

In some embodiments L is a chemical bond. Accordingly, the fusion protein may have the structure A-B wherein subunit A is directly connected to subunit B by a chemical bond.

In some embodiments L is a linker selected from -R¹-[O(CH₂)ₘ]ₙ-R²-, wherein m and n are independently 0 to 10, and R¹ and R² are independently selected from the group: covalent bond, C1-C10 alkylene, -OR³-, C1-C10 polyether, or -O-; and wherein R³ is C1-C5 alkylene.

In some embodiments L may be a linker comprising repeats of glycine and serine residues.

Subunit A may comprise SEQ ID NO:1.

Subunit B may comprise SEQ ID NO:2.

In some embodiments subunit A is SEQ ID NO:1, L is a chemical bond, and subunit B is SEQ ID NO:2.

Whilst the claimed species are fusion proteins, the following definitions are provided for the avoidance of any doubt and apply to the claimed fusion protein.

As used herein, the term "protein" can be used interchangeably with "peptide" or "polypeptide", and means at least two covalently attached alpha amino acid residues linked by a peptidyl bond. The term protein encompasses purified natural products, or chemical products, which may be produced partially or wholly using recombinant or synthetic techniques. The term protein may refer to a complex of more than one polypeptide, such as a dimer or other multimer, a fusion protein, a protein variant, or derivative thereof. The term also includes modified proteins, for example, a protein modified by glycosylation, acetylation, phosphorylation, pegylation, ubiquitination, and so forth. A protein may comprise amino acids not encoded by a nucleic acid codon.

The term "similarity" refers to a degree of similarity between proteins in view of differences in amino acids, but which different amino acids are functionally similar in view of almost equal size, lipophilicity, acidity, etc. is taken into account. A percentage similarity can be calculated by optimal alignment of the sequences using a similarity-scoring matrix such as the Blosum62 matrix described in Henikoff S. and Henikoff J.G., P.N.A.S. USA 1992, 89: 10915-10919. Calculation of the percentage similarity and optimal alignment of two sequences using the Blosum62 similarity matrix and the algorithm of Needleman and Wunsch (J. Mol. Biol. 1970, 48: 443-453) can be performed using the GAP program of the Genetics Computer Group (GCG, Madison, Wl, USA) using the default parameters of the program.

Exemplary parameters for amino acid comparisons in the present invention use the Blosum62 matrix (Henikoff and Henikoff, supra) in association with the following settings for the GAP program:
- Gap penalty: 8
- Gap length penalty: 2
- No penalty for end gaps.

Variants of the fusion proteins that also form part of the present invention are natural or synthetic variants that may contain variations in the amino acid residue sequence due to deletions, substitutions, insertions, inversions or additions of one or more amino acid residues in said sequence or due to an alteration to a moiety chemically linked to a protein. For example, a protein variant may be an altered carbohydrate or PEG structure attached to a protein. The fusion proteins of the invention may include at least one such protein modification.

Substitutional variants of proteins are those in which at least one amino acid residue in the amino acid sequence has been removed and a different amino acid residue inserted in its place. The fusion proteins of the present invention can contain conservative or non-conservative substitutions.

The term "conservative substitution", relates to the substitution of one or more amino acid residues for amino acid residues having similar biochemical properties. Typically, conservative substitutions have little or no impact on the activity of a resulting protein. For example, a conservative substitution in a fusion protein of the present invention may be an amino acid residue substitution that does not substantially affect the ability of the fusion protein to bind to, and activate, CFH. Screening of variants of the fusion proteins of the present invention can be used to identify which amino acid residues can tolerate an amino acid residue substitution. In one example, the relevant biological activity of a modified protein is not decreased by more than 25%, preferably not more than 20%, especially not more than 10%, compared with the fusion protein of the present aspect, for example, when one or more conservative amino acid residue substitutions are effected.

One or more conservative substitutions can be included in a fusion protein of the present invention. In one example, 10 or fewer conservative substitutions are included in the fusion protein. A fusion protein of the invention may therefore include 1, 2, 3, 4, 5, 6, 7, 8, 9, 10 or more conservative substitutions. A polypeptide can be produced to contain one or more conservative substitutions by manipulating the nucleotide sequence that encodes that polypeptide using, for example, standard procedures such as site-directed mutagenesis, gene synthesis, or PCR. Alternatively, a polypeptide can be produced to contain one or more conservative substitutions by using peptide synthesis methods, for example as known in the art.

Examples of amino acid residues which may be substituted for an original amino acid residue in a protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; lie or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val. In one embodiment, the substitutions are among Ala, Val Leu and Ile; among Ser and Thr; among Asp and Glu; among Asn and Gln; among Lys and Arg; and/or among Phe and Tyr. Further information about conservative substitutions can be found in, among other locations, Ben-Bassat et al., (J. Bacteriol. 169:751-7, 1987), O'Regan et al., (Gene 77:237-51, 1989), Sahin-Toth et al., (Protein Sci. 3:240-7, 1994), Hochuli et al., (Bio/Technology 6:1321-5, 1988), WO 00/67796 (Curd et al.) and in standard textbooks of genetics and molecular biology.

Other variants can be, for example, functional variants such salts, amides, esters, and specifically C-terminal esters, and N-acyl derivatives. Also included are peptides which are modified in vivo or in vitro, for example by glycosylation, amidation, carboxylation or phosphorylation.

Fusion proteins according to the present invention can be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula CONR4R5 wherein R4 and R5 are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains may be converted to alkoxy or ester groups, for example C1-C6 alkoxy or C1-C6 alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability.

In a second aspect there is provided a fusion protein comprising SEQ ID NO:3. SEQ ID NO:3 is provided below:

SEQ ID NO:3
DAHKSEVAHRFKDLGEENFKALVLIAFAQYLQQCPFEDHVKLVNEVTEFAKTCVADESAE
NCDKSLHTLFGDKLCTVATLRETYGEMADCCAKQEPERNECFLQHKDDNPNLPRLVRPEV
DVMCTAFHDNEETFLKKYLYEIARRHPYFYAPELLFFAKRYKAAFTECCQAADKAACLLP

KLDELRDEGKASSAKQRLKCASLQKFGERAFKAWAVARLSQRFPKAEFAEVSKLVTDLTK
VHTECCHGDLLECADDRADLAKYICENQDSISSKLKECCEKPLLEKSHCIAEVENDEMPA
DLPSLAADFVESKDVCKNYAEAKDVFLGMFLYEYARRHPDYSVVLLLRLAKTYETTLEKC
CAAADPHECYAKVFDEFKPLVEEPQNLIKQNCELFEQLGEYKFQNALLVRYTKKVPQVST
PTLVEVSRNLGKVGSKCCKHPEAKRMPCAEDYLSVVLNQLCVLHEKTPVSDRVTKCCTES
LVNRRPCFSALEVDETYVPKEFNAETFTFHADICTLSEKERQIKKQTALVELVKHKPKAT
KEQLKAVMDDFAAFVEKCCKADDKETCFAEEGKKLVAASQAALGLSERDKARKEVEEYVK
KIVGESYAKSTKKRHTITVALVNELNNIKNEYLNKIVESTSESQLQILMMESRSKVDEAVSKF
EK

The underlined sequence corresponds to SEQ ID NO:2 and the remaining sequence corresponds to SEQ ID NO:1.

According to third aspect there is provided a medical device, wherein the surface of the medical device is at least partially coated with the fusion protein of the first aspect or the second aspect.

In some embodiments substantially all of the contacting surfaces of the medical device are coated with the fusion protein. The contacting surfaces may correspond to those surfaces of the medical device that contact the tissue or bodily fluid during use.

The medical device can be essentially any medical device which is exposed to tissues or fluids of the body of a subject, e.g. microcapsules for stem cell delivery, stents, stent grafts, orthopaedic implants, catheters, guide wires, heart valve repair devices and extracorporeal circulatory devices.

The present aspect is particularly appropriate to implantable medical devices, e.g. microcapsules, stents, vascular grafts, orthopaedic implants, pacemakers and the like, as well as extracorporeal circulatory devices such as equipment used in haemodialysis, plasmaphoresis, extracorporeal membrane oxygenation, ventricular assist device (VAD) and related procedures.

The medical device may be configured to be incorporated into an extracorporeal circuit. For example, the medical device may comprise tubing that forms a part of an extracorporeal circuit and the contacting surfaces of the medical device may correspond to the internal surfaces of the tubing. The medical device may be a filter cartridge and the contacting surfaces may correspond to the filter medium surfaces of the filter cartridge.

The filter medium or filter medium surfaces may comprise at least 0.1 mg/m² of the fusion protein of the first aspect or the second aspect as measured in physiological conditions. The filter medium or filter medium surfaces may comprise at least 0.2 mg/m² of the fusion protein of the first aspect or the second aspect as measured in physiological conditions. The filter medium or filter medium surfaces may comprise at least 0.3 mg/m² of the fusion protein of the first aspect or the second aspect as measured in physiological conditions. The filter medium or filter medium surfaces may comprise at least 0.4 mg/m² of the fusion protein of the first aspect or the second aspect as measured in physiological conditions. The filter medium or filter medium surfaces may comprise at least 0.5 mg/m² of the fusion protein of the first aspect or the second aspect as measured in physiological conditions. The filter medium or filter medium surfaces may comprise at least 0.6 mg/m² of the fusion protein of the first aspect or the second aspect as measured in physiological conditions. The filter medium or filter medium surfaces may comprise at least 0.7 mg/m² of the fusion protein of the first aspect or the second aspect as measured in physiological conditions. The filter medium or filter medium surfaces may comprise at least 0.8 mg/m² of the fusion protein of the first aspect or the second aspect as measured in physiological conditions. The filter medium or filter medium surfaces may comprise at least 0.9 mg/m² of the fusion protein of the first aspect or the second aspect as measured in physiological conditions. The filter medium or filter medium surfaces may comprise at least 1.0 mg/m² of the fusion protein of the first aspect or the second aspect as measured in physiological conditions.

The filter medium or the filter medium surfaces may comprise from 0.1 mg/m² to 2.0 mg/m² of the fusion protein according to the first aspect or the second aspect as measured under physiological conditions. The filter medium or the filter medium surfaces may comprise from 0.2 mg/m² to 2.0 mg/m² of the fusion protein according to the first aspect or the second aspect as measured under physiological conditions. The filter medium or the filter medium surfaces may comprise from 0.3 mg/m² to 2.0 mg/m² of the fusion protein according to the first aspect or the second aspect as measured under physiological conditions. The filter medium or the filter medium surfaces may comprise from 0.4 mg/m² to 2.0 mg/m² of the fusion protein according to the first aspect or the second aspect as measured under physiological conditions. The filter medium or the filter medium surfaces may comprise from 0.5 mg/m² to 2.0 mg/m² of the fusion protein according to the first aspect or the second aspect as measured under physiological conditions. The filter medium or the filter medium surfaces may comprise from 0.6 mg/m² to 2.0 mg/m² of the fusion protein according to the first aspect or the second aspect as measured under physiological conditions. The filter medium or the filter medium surfaces may comprise from 0.7 mg/m² to 2.0 mg/m² of the fusion protein according to the first aspect or the second aspect as measured under physiological conditions. The filter medium or the filter medium surfaces may comprise from 0.8 mg/m² to 2.0 mg/m² of the fusion protein according to the first aspect or the second aspect as measured under physiological conditions. The filter medium or the filter medium surfaces may comprise from 0.9 mg/m² to 2.0 mg/m² of the fusion protein according to the first aspect or the second aspect as measured under physiological conditions. The filter medium or the filter medium surfaces may comprise from 1.0 mg/m² to 2.0 mg/m² of the fusion protein according to the first aspect or the second aspect as measured under physiological conditions.

Adsorption of proteins to the surfaces of implantable medical devices exposed to blood and consequent complement activation is a common problem and drives inflammatory reactions. By coating at least a portion of the surface of such a medical device with fusion proteins according to the first aspect or the second aspect, complement activity/activations associated with such a surface would be expected to be reduced. The fusion protein would recruit factor H from the blood/serum and decrease complement activation on or associated with the surface.

The medical device may be coated with a composite of the fusion protein of the first aspect or the second aspect bound to CFH.

The medical device may be an implantable medical device or a microcapsule.

The medical device may be a filter medium. The filter medium may be part of a filter cartridge suitable for use in filtration or processing of a bodily fluid.

The medical device may be configured to be used in haemodialysis.

In a fourth aspect there is provided the fusion protein of the first aspect or second aspect for use in the treatment or prevention of a disease, condition or other medical complication associated with aberrant complement regulatory activity in a subject or where reducing complement activity is beneficial.

The disease or condition or other medical complication may be an acute disease, condition or other medical complication.

The disease or condition or other medical complication may be related to complement initiated inflammation. The fusion protein of the first aspect and the second aspect has been shown to reduce complement activation, known to initiate inflammation mediated by cells of the immune system such as neutrophils, macrophages, and other lymphocytes.

The disease, condition or other medical condition are associated with acute complement-mediated inflammation and may be one or more of: Sepsis; Traumatic brain injury; Ischaemic reperfusion injury, transplant-associated thrombotic microangiopathies.

According to a fifth aspect there is provided a recombinant vector comprising a polynucleotide according to the first aspect or the second aspect. Suitable vectors include bacterial or yeast plasmids, cosmids, phagemids, fosmids, wide host-range plasmids and vectors derived from combinations of plasmid and phage or virus DNA. An origin of replication and/or a dominant selection marker can suitably be present in the vector.

The vectors according to the invention are suitable for transforming a host cell. Examples of suitable cloning vectors are plasmid vectors such as pBR322, the various pUC, pEMBL and Bluescript plasmids, or viral vectors.

When used for the expression of a gene encoding the fusion proteins of the first aspect or the second aspect, a vector according to the present aspect typically comprises an expression control sequence operably linked to the nucleic acid sequence coding for the fusion protein to control expression of the relevant polynucleotide. Such expression control sequences generally comprise a promoter sequence and additional sequences which regulate transcription and translation and/or enhance expression levels. Suitable expression control sequences are well known in the art and include eukaryotic, prokaryotic, or viral promoter or poly-A signal. Expression control and other sequences will, of course, vary depending on the host cell selected and can be constitutive or can be made inducible. Examples of useful promoters are the SV-40 promoter (Science 1983, 222: 524-527), the metallothionein promoter (Nature 1982, 296: 39-42), the heat shock promoter (Voellmy et al., P.N.A.S. USA 1985, 82: 4949-4953), the PRV gX promoter (Mettenleiter and Rauh, J. Virol. Methods 1990, 30: 55-66), the human CMV IE promoter (US 5,168,062), the Rous Sarcoma virus LTR promoter (Gorman et al., P.N.A.S. USA 1982, 79: 6777-6781), or human elongation factor 1 alpha or ubiquitin promoter. Many other suitable control sequences are known in the art, and it would be routine for the skilled person to select suitable sequences for the expression system being used.

After the polynucleotide has been cloned into an appropriate vector, the construct may be transferred into a cell (e.g. animal cell, bacteria, or yeast) by means of an appropriate method, such as electroporation, CaCl₂ transfection or lipofectins. When a baculovirus expression system is used, the transfer vector containing the polynucleotide may be transfected together with a complete baculo genome.

These techniques are well known in the art and the manufacturers of molecular biological materials (such as Clontech, Stratagene, Promega, and/or Invitrogen) provide suitable reagents and instructions on how to use them. Furthermore, there are a number of standard reference text books providing further information on this, e.g. Rodriguez, R. L. and D. T. Denhardt, ed., "Vectors: A survey of molecular cloning vectors and their uses", Butterworths, 1988; Current protocols in Molecular Biology, eds.: F. M. Ausubel et al., Wiley N. Y. , 1995; Molecular Cloning: a Laboratory Manual, supra; and DNA Cloning, Vol. 1-4, 2nd edition 1995, eds.: Glover and Hames, Oxford University Press).

In a sixth aspect there is provided a cell capable of expressing a recombinant protein such as the fusion protein of the first aspect or the second aspect, characterised in that the cell comprises a polynucleotide according encoding the recombinant protein to be expressed. Suitably the cell is a host cell transformed with a polynucleotide or vector as described above. The polynucleotide or vector according to the invention can be stably integrated into the genomic material of the cell or can be part of an autonomously replicating vector. "Recombinant" in this context refers to a protein that is not expressed in the cell in nature.

Accordingly, the cell may be capable of producing a recombinant protein.

Host cells can be cultured in conventional nutrient media which can be modified, e.g. for appropriate selection, amplification or induction of transcription and thus expression of the recombinant protein.

The host cells can be prokaryotic or eukaryotic. Suitable prokaryotic cells include, for example, *E. coli*, *Corynebacterium* or *Pseudomonas fluorescens.* Suitable eukaryotic cells include, for example, *Pichia pastoris*, insect cells, HeLa, BHK, HEK-293T, CHO, or COS-7 cells.

Suitable culture conditions for various suitable cell types are well-known to the person skilled in the art.

According to a sixth aspect there is provided a fusion protein according to the first aspect or the second aspect for use in the treatment or prevention of a disease (condition) or other medical complication associated with aberrant complement regulatory activity in a subject or where reducing complement activity is beneficial.

Fusion proteins of the first aspect or the second aspect are particularly useful in treating diseases in which complement is inappropriately active, e.g. is overactive.

Fusion proteins of the first aspect or the second aspect are particularly useful in treating disease in which the alternative complement pathway is inappropriately active.

Fusion proteins of the first aspect or the second aspect are particularly useful in treating disease in which CFH is unable to adequately modulate complement activation, e.g. because of lowered levels of CFH in plasma or mutation or a SNP within the gene (or its regulatory regions) that encodes CFH which reduces CFH production or regulatory activity, or because of changes in self surface markers as may occur e.g. with age or pathology, or because of competition with surface-binding by CFH from CFH-related proteins. Additionally, fusion proteins of the first aspect or second aspect can be useful where the regulatory mechanisms of complement have been overrun for some other reason, e.g. where other complement regulators are deficient in some respect or when triggers of complement activation - that include both foreign matter and the products of host cell damage or death - are abundant. Such diseases are well known to those familiar with the field. As is known in the art, CFH regulates complement activation in fluid phase, and on self-cells and surfaces, by possessing both cofactor activity for the factor I-mediated C3b cleavage, and decay-accelerating activity against the alternative pathway C3-convertase, C3b.Bb.

The disease or condition or other medical complication may be an acute disease, condition or other medical complication.

The disease or condition or other medical complication may be related to complement initiated inflammation. The fusion protein of the first aspect and the second aspect has been shown to reduce complement activation, which initiates inflammation mediated by cells such as neutrophils, macrophages, and other lymphocytes.

The disease, condition or other medical condition may be one or more of: ; Sepsis; Traumatic brain injury; Ischaemic reperfusion injury or Transplant-associated Thrombotic Microangiopathies.

The disease mentioned above may be associated with a mutation in or affecting CFH. However, some of the diseases mentioned above (e.g. PNH) do not involve mutations or SNPs in CFH, and, for those disease that do involve mutations or SNPs in CFH, not all occurrences of the diseases mentioned above are caused (entirely or in part) by such mutations or SNPs in CFH. The fusion proteins of the first aspect or the second aspect are clearly well adapted for cases where CFH is directly involved. However, even in cases where a mutation or SNP in CFH is not present, the fusion proteins of the first aspect or the second aspect can still be useful in treating the condition.

The fusion proteins of the first aspect or the second aspect enhance the activity of CFH and, as such, can be used to augment the activity of CFH in conditions where inadequate activity of CFH contributes to the pathology of the condition.

In view of the advent of stratified/personalised medicine, in which diseases are profiled and appropriate therapies selected based upon the profile, the fusion proteins of the first aspect or the second aspect can be administered where a disease, for example one of the diseases mentioned above, involves aberrant CFH levels or activity or in which the alternative complement pathway is overactive. This allows treatment with the fusion proteins of the first aspect or the second aspect to be focussed on conditions where a positive effect is likely to occur.

In a seventh aspect there is provided a pharmaceutical composition comprising the fusion protein of the first aspect or the second aspect or a nucleic acid encoding a fusion protein of the first aspect or the second aspect.

The pharmaceutical composition may comprise a pharmaceutically acceptable carrier.

As used herein "pharmaceutically-acceptable carrier" includes any and all solvents, dispersion media, coatings, antibacterial and antifungal agents, isotonic and absorption delaying agents, and the like that are physiologically compatible. In one embodiment, the carrier is suitable for parenteral administration. The carrier can be suitable for intravenous, subcutaneous, intraperitoneal or intramuscular administration. In another embodiment, the carrier is suitable for oral administration. Pharmaceutically-acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the fusion proteins of the disclosure, use thereof in the pharmaceutical compositions of the disclosure is contemplated. The composition, if desired, can also contain minor amounts of wetting or emulsifying agents, or pH buffering agents. Supplementary active compounds can also be incorporated into the compositions.

Ordinarily, the preparation of such pharmaceutical compositions entails combining the active agent with buffers, antioxidants such as ascorbic acid, low-molecular weight (less than about 10 residues) polypeptides, proteins, amino acids, carbohydrates including glucose, sucrose or dextrins, chelating agents such as EDTA, glutathione and other stabilizers and/or excipients. Neutral buffered saline or saline mixed with conspecific serum albumin are exemplary appropriate diluents for protein therapeutics.

The composition may be formulated as a lyophilisate using appropriate excipient solutions (e.g., sucrose) as diluents.

The compositions can take the form of solutions, suspensions, emulsions, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc.

Various literature references are available to facilitate the selection of pharmaceutically acceptable carriers or excipients. See, e.g., Remington's Pharmaceutical Sciences and US Pharmacopeia: National Formulary, Mack Publishing Company, Easton, PA (1984); Hardman et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis et al. (Eds.) (1993) Pharmaceutical Dosage Forms: Parenteral Medications, Marcel Dekker, NY; Lieberman, et al. (Eds.) (1990) Pharmaceutical Dosage Forms: Tablets, Marcel Dekker, New York, NY; Lieberman, et al. (Eds.) (1990) Pharmaceutical Dosage Forms: Disperse Systems, Marcel Dekker, NY; Weiner, Wang, E., Int. J. Pharm. 185:129-188 (1999) and Wang W. Int. J. Pharm. 203:1-60 (2000), and Kotkoskie (2000) Excipient Toxicity and Safety, Marcel Dekker, New York, NY.

Such compositions will contain a therapeutically effective amount of the fusion protein of the first aspect or the second aspect, preferably in purified form, together with a suitable amount of carrier so as to provide the form for proper administration to the patient. The formulation should suit the mode of administration.

In some embodiments the fusion protein of the first aspect or the second aspect may be provided as a complex with CFH. Such a complex is particularly valuable where a subject to be treated has reduced ability to produce functioning CFH, and thus there may be inadequate CFH present in the subject for the protein to bind to and activate.

The composition may be formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The compositions of the disclosure may be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and feme hydroxides, etc.

Various delivery systems are known and can be used to administer a fusion protein of the first aspect or the second aspect, e.g., encapsulation in liposomes, micro-particles, and microcapsules: use of recombinant cells capable of expressing the fusion protein of the first aspect or the second aspect, use of receptor-mediated endocytosis (e.g., Wu and Wu, 1987, J. Biol. Chem. 262:4429-4432); construction of a therapeutic nucleic acid as part of a retroviral or other vector, etc.

In another embodiment, the pharmaceutical compositions of the present aspect can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the pharmaceutical compositions of the present aspect can be delivered via a controlled release system.

In a specific embodiment where the pharmaceutical compositions of the present aspect comprise a nucleic acid encoding a fusion protein, the nucleic acid can be administered *in vivo* to promote expression of its encoded fusion protein, by constructing it as part of an appropriate nucleic acid expression vector and administering it so that it becomes intracellular, e.g., by use of a retroviral vector (U.S. Patent No. 4,980,286), or by direct injection, or by use of micro-particle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864- 868), etc. Alternatively, a nucleic acid can be introduced intracellularly and incorporated by homologous recombination within host cell DNA for expression.

The amount of the pharmaceutical compositions of the present aspect which will be effective in the treatment of a particular disorder or condition will depend on the nature of the disorder or condition, and can be determined by standard clinical techniques. Appropriate dosages can be determined in trials. In accordance with appropriate industry standards, preservatives may also be added, such as benzyl alcohol. The amount and frequency of administration will depend, of course, on such factors as the nature and severity of the indication being treated, the desired response, the condition of the patient, and so forth. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 1-500 micrograms of active compound per kilogram body weight. Effective doses may be extrapolated from dose-response curves derived from *in vitro* or animal model test systems.

According to an eighth aspect there is provided a pharmaceutical pack or kit comprising one or more containers filled with one or more of the ingredients of the pharmaceutical compositions of the seventh aspect. Optionally associated with such container(s) can be a notice in the form prescribed by a governmental agency regulating the manufacture, use or sale of pharmaceuticals or biological products, which notice reflects approval by the agency of manufacture, use or sale for human administration.

In a ninth aspect there is provided a method of treatment or prevention of a disease in a subject by administration to a subject of an effective amount of a pharmaceutical composition of the seventh aspect. In particular, the method may provide the treatment or prevention of a disease associated with aberrant complement activity in a subject.

The subject is preferably an animal, preferably a mammal, and most preferably a human.

The disease or condition or other medical complication may be an acute disease, condition or other medical complication.

The disease or condition or other medical complication may be related to complement initiated inflammation. The fusion protein of the first aspect and the second aspect has been shown to reduce complement activation, which initiates inflammation mediated by cells such as neutrophils, macrophages, and other innate lymphocytes.

The disease, condition or other medical condition may be one or more of: ; Sepsis; Traumatic brain injury; Ischaemic reperfusion injury or transplant-associated thrombotic microangiopathies.

The method of treatment or prevention of disease may be by the administration of the fusion protein of the first aspect or the second aspect in the form of a pharmaceutical composition of the seventh aspect to a subject (e.g., a mammal particularly a human) in need thereof.

Methods of introduction include, but are not limited to intradermal, intramuscular, transdermal, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents.

Administration can be systemic or local. Pulmonary administration can be employed, e.g., by use of an inhaler or nebulizer, and formulation with an aerosolizing agent.

Typically, methods of the present aspect may comprise administering a pharmaceutical composition comprising a pharmaceutically effective amount of a fusion protein of the first aspect or second aspect or a composition comprising a nucleic acid capable of expressing a pharmaceutically effective amount of a fusion protein of the first aspect or the second aspect *in vivo.* The pharmaceutically effective amount employed may vary according to factors such as the disease state, age, sex, and weight of the individual.

A pharmaceutically effective amount of a fusion protein of the first aspect or the second aspect may be from about 1 µg protein/1 kg body weight of subject to about 500 mg protein/1 kg body weight of subject, or from about 10 µg protein/1 kg body weight of subject to about 500 mg protein/1 kg body weight of subject, or from about 10 mg protein/1 kg body weight of subject to about 500 mg protein/1 kg body weight of subject, or from about 100 mg protein/1 kg body weight of subject to about 500 mg protein/1 kg body weight of subject, or from about 10 mg protein/1 kg body weight of subject to about 500 mg protein/1 kg body weight of subject, or from about 100 mg protein/1 kg body weight of subject to about 500 mg protein/1 kg body weight of subject, or from about 100 µg protein/1 kg body weight of subject to about 25 mg protein/1 kg body weight of subject, or from about 1 mg protein/1 kg body weight of subject to about 25 mg protein/1 kg body weight of subject, or from about 5 mg protein/1 kg body weight of subject to about 25 mg protein/1 kg body weight of subject, or from about 10 mg protein/1 kg body weight of subject to about 25 mg protein/1 kg body weight of subject, or from about 15 mg protein/1 kg body weight of subject to about 25 mg protein/1 kg body weight of subject, or from about 100 µg protein/1 kg body weight of subject to about 10 mg protein/1 kg body weight of subject, or from about 1 mg protein/1 kg body weight of subject to about 10 mg protein/1 kg body weight of subject, or from about 2.5 mg protein/1 kg body weight of subject to about 10 mg protein/1 kg body weight of subject, or from about 5 mg protein/1 kg body weight of subject to about 10 mg protein/1 kg body weight of subject, or from about 7.5 mg protein/1 kg body weight of subject to about 10 mg protein/1 kg body weight of subject.

In some embodiments, a pharmaceutically effective amount of a fusion protein of the first aspect or the second aspect may be 0.5 mg protein/1 kg body weight of subject, 1 mg protein/1 kg body weight of subject, 2 mg protein/1 kg body weight of subject, 3 mg protein/1 kg body weight of subject, 4 mg protein/1 kg body weight of subject, 5 mg protein/1 kg body weight of subject, 6 mg protein/1 kg body weight of subject, 7 mg protein/1 kg body weight of subject, 8 mg protein/1 kg body weight of subject, 9 mg protein/1 kg body weight of subject, or 10 mg protein/1 kg body weight of subject.

A unit dosage form refers to physically discrete units suited as unitary dosages for the mammalian subjects to be treated; each unit containing a predetermined quantity of the fusion protein of the first aspect or the second aspect calculated to produce the desired therapeutic effect in association with the required pharmaceutical carrier. A unit dosage form of a protein of the invention may be from about 1 mg to about 1000 mg, from about 25 mg to about 1000 mg, from about 50 mg to about 1000 mg, from about 100 mg to about 1000 mg, from about 250 mg to about 1000 mg, from about 500 mg to about 1000 mg, from about 100 mg to about 500 mg, from about 200 mg to about 500 mg, from about 300 to about 500 mg, or from about 400 mg to about 500 mg. A unit dose of a fusion protein of the first aspect or the second aspect may be about 100 mg, 200 mg, 300 mg, 400 mg, 500 mg, 600 mg, or 700 mg.

Compositions of the disclosure may comprise fusion proteins of the first aspect or the second aspect at a level of from about 0.1 wt % to about 20 wt %, from about 0.1 wt % to about 18 wt %, from about 0.1 wt % to about 16 wt %, from about 0.1 wt % to about 14 wt %, from about 0.1 wt % to about 12 wt %, from about 0.1 wt % to about 10 wt %, from about 0.1 wt % to about 8 wt %, from about 0.1 wt % to about 6 wt % from about 0.1 wt % to about 4 wt %, from about 0.1 wt % to about 2 wt %, from about 0.1 wt % to about 1 wt %, from about 0.1 wt % to about 0.9 wt %, from about 0.1 wt % to about 0.8 wt %, from about 0.1, wt % to about 0.7 wt %, from about 0.1 wt % to about 0.6 wt %, from about 0.1 wt % to about 0.5 wt %, from about 0.1 wt % to about 0.4 wt %, from about 0.1 wt % to about 0.3 wt %, or from about 0.1 wt % to about 0.2 wt % of the total weight of the composition.

Pharmaceutical compositions of the disclosure may comprise one or more fusion proteins of the first aspect or the second aspect at a level of from about 1 wt % to about 20 wt %, from about 1 wt % to about 18 wt %, from about 1 wt % to about 16 wt %, from about 1 wt % to about 14 wt %, from about 1 wt % to about 12 wt %, from about 1 wt % to about 10 wt %, from about 1 wt % to about 9 wt %, from about 1 wt % to about 8 wt %, from about 1 wt % to about 7 wt %, from about 1 wt % to about 6 wt %, from about 1 wt % to about 5 wt %, from about 1 wt % to about 4 wt %, from about 1 wt % to about 3 wt %, or from about 1 wt % to about 2 wt % of the total weight of the composition. Pharmaceutical compositions of the disclosure may comprise one or more fusion proteins of the first aspect or the second aspect at a level of about 0.1 wt %, about 0.2 wt %, about 0.3 wt %, about 0.4 wt %, about 0.5 wt %, about 0.6 wt %, about 0.7 wt %, about 0.8 wt %, about 0.9 wt %, about 1 wt %, about 2 wt %, about 3 wt %, about 4 about 5 wt %, about 6 wt %, about 7 wt %, about 8 wt %, or about 9 wt % based on the total weight of the composition.

Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Compositions of the disclosure may be formulated and administered by intravenous, intramuscular, or subcutaneous injection. In some embodiments, compositions of the invention may be administered subcutaneously or intramuscularly.

In some embodiments a dosage regimen may entail administering repeat doses, for example, administering a weekly dose. Treatment regimens may entail a weekly dose for one period of time (for example, for four weeks) followed by a less frequent "maintenance" dosage regimen (for example, one monthly or once bimonthly). Dosage regimens may be adjusted to achieve the desired therapeutic outcomes.

In a tenth aspect there is provided a fusion protein according to the first aspect or the second aspect immobilised on a surface. The surface can be any suitable surface, and includes, for example, the surface of a bead, container, medical device, microcapsule, or a biological tissue.

By attaching the fusion protein to a surface, it permits the surface to bind to CFH. This can be useful in many different applications. For example, the bound CFH can be used to protect the surface from the consequences of C3b amplification. Alternatively, the surface can be used to selectively bind CFH for diagnostic purposes, to extract or purify CFH from a mixture, or to bind CFH to identify molecules that interact and thus bind to the captured CFH.

Typically, the fusion protein binds to the surface via subunit A such that subunit B is available to bind to CFH.

In an eleventh aspect there is provided a method of treating a medical device, the method comprising:
- providing a medical device;
- providing a fusion protein according to the first aspect or second aspect; and
- binding said fusion protein to at least a portion of the surface of the medical device.

Binding of the fusion protein according to the first aspect or second aspect to the medical device can, of course, be carried out by any suitable technique, including those discussed above.

In a twelfth aspect there is provided a method of detecting the presence or quantity of CFH in a sample, the method comprising:
- providing a sample;
- providing a fusion protein according to the first aspect or second aspect;
- contacting the sample with the fusion protein; and
- detecting binding of the fusion protein to the CFH present in the sample.

Suitably the fusion protein could be immobilised on a surface and used to bind to CFH in a method analogous to a conventional "ELISA". The high affinity and selectivity of PspCN (SEQ ID NO:2) makes it an attractive agent for use in quantifying levels of CFH.

The method may suitably be a diagnostic method, and thus can involve detecting the presence of CFH in a biological sample from a subject, e.g. a sample of blood or a fraction of blood such as plasma. For example, CFH and a fusion protein comprising the fusion protein of the first aspect or second aspect to horse radish peroxidase could be allowed to form a complex that is then captured by an antibody and quantified. Alternatively fluorescently labelled fusion protein of the first aspect or second aspect could be used in an assay in which the formation of a complex with CFH is monitored by thermophoresis or another suitable means.

In a further aspect of the present invention, there is provided a method of purifying or separating CFH from a sample, the method comprising:
- providing a fusion protein according to the first aspect or second aspect immobilised on a surface;
- providing a sample;
- passing the sample over the surface; and
- recovering CFH bound to the surface; and/or
- recovering the sample that is devoid or depleted of CFH.

The surface can be, for example, the surface of a container, a conduit, a matrix (e.g. a porous or fibrous matrix), beads, gels, or any packing material used in chromatography.

The surface can be, for example, the surface of a container, a conduit, a matrix (e.g. a porous or fibrous matrix), beads, gels, or any packing material used in chromatography.

In a further aspect of the present invention, there is provided a method of coating implantable microcapsules with fusion proteins according to the first aspect or second aspect comprising the steps:
- providing a suitable microcapsule; and
- immobilising the fusion proteins onto said microcapsule.

Accordingly, microcapsules so coated have a reduced likelihood of complement activation and subsequent rejection. Examples of such microcapsules include, but are not limited to, various alginate microcapsules designed for the encapsulation of cells such as pancreatic islet cells for subsequent implantation into patients with type I diabetes mellitus.

The method may further comprise the step of providing CFH or a functional variant thereof to be bound to the fusion protein coating the microcapsule.

The following definitions are useful for understanding the proper scope of the present invention:
"Amino acid sequence", as used herein, refers to an oligopeptide, peptide, polypeptide, or protein sequence, and fragments or portions thereof, and to naturally occurring or synthetic molecules. "Amino acid sequence" and like terms, such as "polypeptide" or "protein" as recited herein are not meant to limit the amino acid sequence to the complete, native amino acid sequence associated with the recited protein molecule.

"Cell culture", as used herein, refers to a proliferating mass of cells that may be in either an undifferentiated or differentiated state.

"Fusion protein", as used herein, refers to a protein containing amino acid sequences from each of two distinct proteins; it is formed by the expression of a recombinant gene in which two coding sequences have been joined together such that their reading frames are in phase. Hybrid genes of this type may be constructed in vitro in order to label the product of a particular gene with a protein that can be more readily assayed (for example, a gene fused with lacZ in *E. coli* to obtain a fusion protein with β-galactosidase activity). Alternatively, a protein may be linked to a signal peptide to allow its secretion by the cell. Alternatively, a protein may be linked to a peptide to facilitate purification (e.g. a polyhistidine-tag).

Alternatively, a protein may be linked to a peptide to improve expression in a host cell (e.g. fusions with SUMO proteins).

The term "isolated" means a biological component (such as a nucleic acid molecule or protein) that has been substantially separated or purified away from other biological components in the cell of the organism in which the component naturally occurs, i.e., other chromosomal and extra chromosomal DNA and RNA, and proteins. Nucleic acids and proteins that have been "isolated" include nucleic acids and proteins purified by standard purification methods. The term also embraces nucleic acids and proteins prepared by recombinant expression in a host cell as well as chemically synthesized nucleic acids, proteins and peptides.

"Nucleic acid' or 'nucleic acid sequence", as used herein, refers to a polymer of nucleotides in which the 3' position of one nucleotide sugar is linked to the 5' position of the next by a phosphodiester bridge. In a linear nucleic acid strand, one end typically has a free 5' phosphate group, the other a free 3' hydroxyl group. Nucleic acid sequences may be used herein to refer to oligonucleotides, or polynucleotides, and fragments or portions thereof, and to DNA or RNA of genomic or synthetic origin that may be single- or double-stranded, and represent the sense or antisense strand.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are mentioned in this application, and which are open to public inspection, and the contents of all such papers and documents are incorporated herein by reference in their entirety.

### Brief Description of the Figures

Embodiments of the present invention will now be described, by way of non-limiting example, with reference to the accompanying drawings.

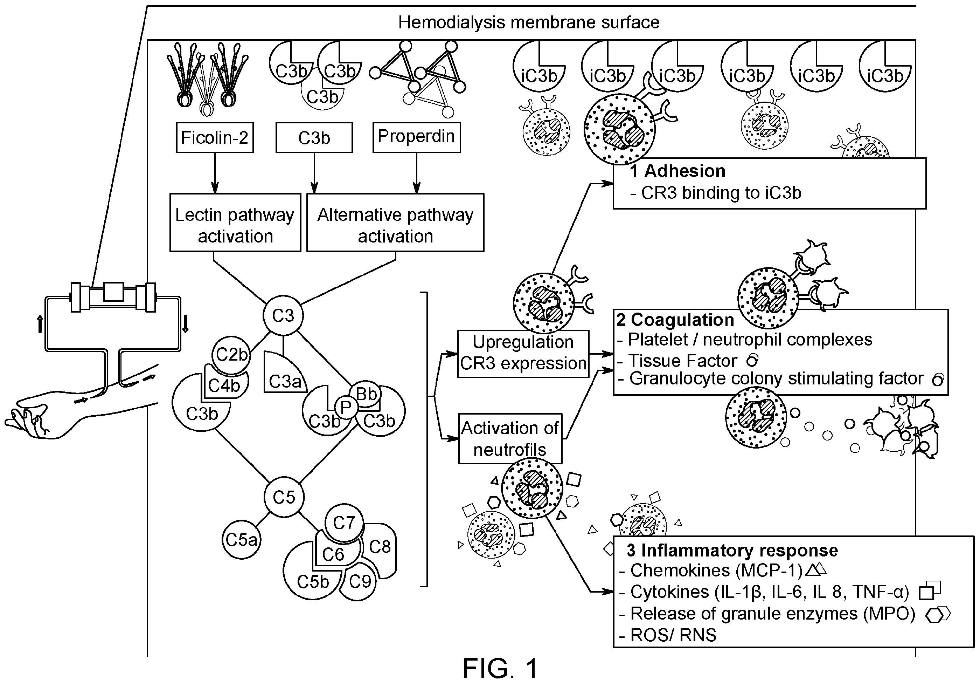

**Figure 1****:** Model for Complement Activation in Haemodialysis (Poppelaars et al. 2018) The principal mechanism of action leading to complement activation in haemodialysis is the binding of ficolin-2 to the membrane resulting in lectin pathway activation. Simultaneously, properdin and/or C3b bind to the membrane resulting in alternative pathway activation. Complement activation results in the formation of anaphylatoxins (C3a, C5a), opsonins (C3b, iC3b) and the membrane attack complex (C5b-9). First, complement activation leads to the upregulation of complement receptor 3 (CR3) allowing leukocytes to bind C3 fragments deposited on the membrane, leading to leukopenia. Second, CR3 on neutrophils is also important for the formation of platelet-neutrophil complexes, which contributes to thrombotic processes. Furthermore, C5a generation during haemodialysis leads to the expression of tissue factor and granulocyte colony-stimulating factor in neutrophils, shifting haemodialysis patients to a procoagulant state. Third, complement activation also promotes recruitment and activation of leukocytes resulting in the oxidative burst and the release of pro-inflammatory cytokines and chemokine's. More specifically, the activation of neutrophils by C5a leads to the release of granule enzymes, e.g., myeloperoxidase (MPO);
**Figure 2****:** A proposed model of the FP1 (H-Guard) fusion protein. PspCN (SEQ ID NO:2) circled (4K12.pdb), HuSA (SEQ ID NO:1) grey (1A06.pdb);
**Figure** 3: Circular dichroism analysis of the secondary structure of FP1 in solution would suggest that the secondary structure of the fusion protein is essentially the same as the constituent parts. FP1 is very well folded and predominantly alpha helical in solution. The CD spectrum is typical of a protein existing in an alpha helical form;
**Figure 4****:** Mass of full-length FP1 calculated by MaxEnt (Waters Corporation) from the intact protein charge state distribution with a narrow range of 0.1 Da. Calculated mass assuming 17 intact di-sulphide bonds: 75697.74 Da. Experimentally determined intact mass 75698 ± 4Da;
**Figure 5****:** Plasmid map of the FP1 Zeocin-resistance expression vector, with linearization site Bglll;
**Figure 6****:** Plasmid map of the FH9 (N529Q) Kanamycin / Geneticin-resistance expression vector with linearization site Bglll;
**Figure 7****:** 6x priming using Fresenius FX60 dialysis filters Quantitation results;
**Figure 8****:** 6x priming using Fresenius FX60 dialysis filters load per filter, based on the FP1 load and the dialysis surface area for an FX60 filter;
**Figure 9****:** FP1 loading/m² of dialysis filter surface area for 2x FX60 and 2x Nipro Elisio-15H dialysis filters;
**Figure 10****:** FP1 antigen staining for 3x FX60 and 2x Elisio-15H dialysis filters used in the above experiment. For the FX60, a single filter was primed with Human Serum Albumin and for the Nipro filters the no coat filter was similarly treated but without any priming. DAB colour development results in a distinctive pink coloration on the FP1 primed filters;
**Figure 11****:** FP1 priming of FX100 filters on 6008 dialysis machine FP1 quantitation of sampling from port A (4 runs) and B (2 runs);
**Figure 12****:** FP1 Antigen staining for one of the filters vs an uncoated FX100 as control;
**Figure 13****:** FP1 quantitation in 6x50 ml fractions collected during priming;
**Figure 14****:** TCC/C5b-9 levels in time course samples from 2x FP1 primed and 2x uncoated AN69ST filters. P value at 4hrs determined using T test;
**Figure 15****:** Ave C3a for 2xFP1 coated vs uncoated AN69ST filters. Duplicate samples were analysed in duplicate (n=4) and compared using T Tests at the various time points;
**Figure 16****:** Ave C5a for 2xFP1 coated vs uncoated AN69ST filters. Duplicate samples were analysed in duplicate (n=4) and compared using T Tests at the various time points;
**Figure 17****:** FP1 ("A0T") (upper panel) and FH (lower panel) antigen staining of AN69ST filters after FP1 priming and 4hrs 50% human serum circulation. For background staining a single HuSA primed filter was used. Note the strong DAB FP1 and FH antigen staining vs the DAB background on the AN69ST filters;
**Figure 18****:** A schematic representation of an example haemodialysis circuit;
**Figure 19****:** A plot of C5b9 concentration as a function of time for baseline/control systems and the test system treated with FP1 ("H-Guard");
**Figure 20****:** A plot of C5b9 concentration as a function of time for a first, "baseline", dialysis with non coated filters, a second subsequent dialysis with filters treated with FP1 ("H Guard") and a third dialysis ("Follow-Up") with non-treated filters.

### Detailed Description

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

### Example 1

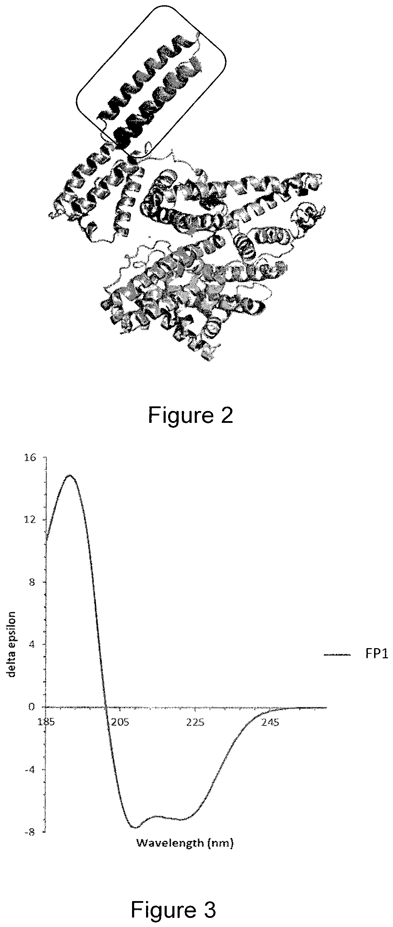

An example fusion protein (FP1) comprising the sequence SEQ ID NO:3 is a 665 amino acid fusion protein between PspCN and human albumin, expressed as a single protein. The FP1 fusion protein has a theoretical molecular weight of 75731.77 Da (75697.74 Da assuming 17 intact di-sulphide bonds). The primary (amino acid) structure of the mature protein is shown in Figure 2 and Figure 3.

PspCN is connected to the C-terminus of human serum albumin by a peptide bond. FP1 is expressed in yeast as a single chain.

Mass spec analysis confirmed the molecular size of the purified yeast expressed FP1. Full-length mass determination of FP1, 1 mg/mL drug product in DPBS solution was determined on a Waters Xevo G2TOF mass spectrometer after on-line desalting.

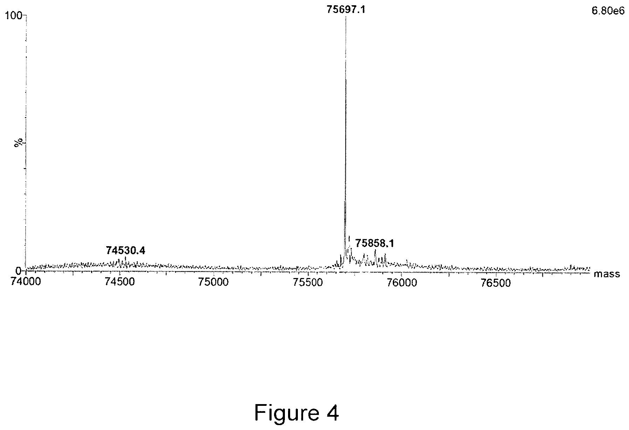

The mass spectrum of FP1 is characteristic of a single species and confirms the predicted mass from sequence. The predicted mass, assuming 17 intact di-sulphide bonds, is 75697.74 Da. Experimentally determined intact mass was 75698 ± 4Da (an uncertainty of 4 Da is acceptable for a single protein species of 76 kDa on this instrument). Representative data is shown in Figure 4.

### Complement Activation During Haemodialysis and the Role of FP1

Notwithstanding advances in dialyzer and haemodialysis technology, cardiovascular morbidity and mortality, due to chronic inflammation remains extremely high in patients undergoing chronic haemodialysis.

One factor thought to be involved, is an immunological incompatibility between the dialyzer filter membrane (which is ultimately a foreign surface) and the patient's own blood driven by complement activation. Short-term effects of complement activation include promoting inflammation and coagulation Poppelaars F, Faria B, Gaya da Costa M, Franssen CFM, van Son WJ, Berger SP, Daha MR, Seelen MA. The Complement System in Dialysis: A Forgotten Story? Front Immunol. 2018 Jan 25;9:71. doi: 10.3389/fimmu.2018.00071. PMID: 29422906; PMCID: PMC5788899.

Indeed, an important adverse event in dialysis is the "first-use syndrome," named after the fact that these reactions are most severe with patients new to dialysis treatment. This incompatibility reaction is the result of complement activation by the membrane and closely resembles the pseudo-anaphylactic clinical picture that is also known as complement activation-related pseudoallergy (CARPA) (Hempel et al. 2017; Szebeni 2014). Hempel JC, Poppelaars F, Gaya Da Costa M, Franssen CF, de Vlaam TP, Daha MR, et al. Distinct in vitro complement activation by various intravenous iron preparations. Am J Nephrol (2017) 45:49-59.10.1159/000451060; Szebeni J. Complement activation-related pseudoallergy: a stress reaction in blood triggered by nanomedicines and biologicals. Mol Immunol (2014) 61:163-73.10.1016/j.molimm.2014.06.038

Over the past decades, dialyzer membranes have been developed with improved biocompatibility. Nonetheless, even with modern "biocompatible" dialyzer membranes significant complement activation still occurs (Hempel et al. 2017; Poppelaars et al. 2016; Lines et al. 2016). Poppelaars F, Gaya da Costa M, Berger SP, Assa S, Meter-Arkema AH, Daha MR, et al. Strong predictive value of mannose-binding lectin levels for cardiovascular risk of hemodialysis patients. J Transl Med (2016) 14:236.10.1186/s12967-016-0995-5; Lines SW, Richardson VR, Thomas B, Dunn EJ, Wright MJ, Carter AM. Complement and Cardiovascular Disease - The Missing Link in Haemodialysis Patients? Nephron 2016;132:5-14

During a single haemodialysis session soluble C5b-9 (sC5b-9) levels and C3d/C3-ratios in the plasma are reported to increase by up to 70% (Hempel et al. 2017; Poppelaars et al. 2016).

In addition to the short-term complications, long-term complications of dialysis, such as infection, fibrosis and cardiovascular events, have also been linked to activation of the complement system (Poppelaars et al. 2018).

An overview of Complement activation mechanisms, with respect to haemodialysis is shown in Figure 1.

FP1 has been designed to not only provide a solution that can be used for the primary purpose of priming the haemodialysis blood sets and dialysers, but additionally incorporates an ancillary medicinal component that may significantly reduce complement activation, and thereby improve short and long-term clinical outcomes in this patient population.

The PspCN peptide component (corresponding to SEQ ID NO:2) of FP1 is designed to target human Factor H and binds to Factor H with high affinity as described in International patent application WO 2015/055991 in the name of the University Court of the University of Edinburgh, which is incorporated herein by reference in its entirety. Once bound, the complex down regulates complement activity by interfering with the complement cascade. The human serum albumin component (corresponding to SEQ ID NO:1) of FP1 allows coating of surfaces, particularly those of extra-corporeal devices such as haemodialysis filters. Anchoring PspCN to the internal surfaces of devices will expose PspCN to circulating blood components and it will complex with Factor H and downregulate complement activation on the device surface.

Previous studies have suggested that the innate immune system plays a key role in the development of cardiovascular disease in haemodialysis patients. Cardiovascular complications are caused largely by activation of the patient's blood-borne complement system which recognises the dialyser as a large foreign object and initiates inflammatory and coagulatory processes. This results in proinflammatory molecules being pumped back into the patient (via the dialysis circuit) causing stress and damage to cardiovascular health (Ekdahl et al, 2017 Ekdahl, K., Soveri, I., Hilborn, J. et al. Cardiovascular disease in haemodialysis: role of the intravascular innate immune system. Nat Rev Nephrol 13, 285-296 (2017). https://doi.org/10.1038/nrneph.2017.17). Although damage from a single dialysis session is slight, the repetitive (as in chronic kidney disease) or prolonged (as in acute kidney disease) nature of the procedures leads to more profound damage and a high mortality rate from cardiovascular disease.

Despite convincing evidence supporting the role of complement activation as a cause of haemodialysis 'bio-incompatibility', at present, there are no targeted treatment options available for these patients. Existing complement inhibitors have not yet proven to be a feasible option in this area predominantly due to concerns of systemic side effects that would inevitably be seen with repetitive exposure. FP1 overcomes this as it is a complement modulator acting on the extracorporeal circuit the patient is exposed to and therefore prevents complications associated with long-term immunosuppression.

Factor H (FH) is a key down-regulator of complement activation which protects host cells from self-attack by the complement system. In an attempt to evade annihilation by the mammalian complement system, some microbes capture FH to protect themselves against host-mediated complement attack, by using either specific receptors and/or molecular mimicry to appear more like a host cell.

PspCN, derived from the N-terminal domain of *Streptococcus pneumoniae* protein PspC, binds to FH with extraordinarily high affinity and holds it in a previously uncharacterised conformation (Herbert AP, Makou E, Chen ZA, Kerr H, Richards A, Rappsilber J & Barlow, PN 2015, 'Complement Evasion Mediated by Enhancement of Captured Factor H: Implications for Protection of Self-Surfaces from Complement' Journal of Immunology, vol. 195, no. 10, pp. 4986-98). This conformational change of FH doubles its affinity for C3b and increases 5-fold the ability of FH to accelerate the decay of the binary enzyme complex (C3bBb) responsible for converting C3 to C3b in the amplification loop. Thus, FP1 coated on an extra-corporeal device such as a haemodialysis filter acts to cloak the "foreignness" of the devices and to the patient's immune system it appears as self and activation of complement is inhibited.

Therefore, FP1 adheres to the inner coatings of the blood tubing sets and dialyser via the albumin portion (SEQ ID NO:1) whilst the PspCN part of the molecule (SEQ ID NO:2) provides a means to improve the immunological biocompatibility of the extracorporeal system, by binding to Factor H.

### Methods of Making FP1

### 1) Expression Plasmids

FP1 protein was co-expressed in *Komagatella phaffii* (formerly known as *Pichia pastoris*) along with a binding partner FH9 peptide (N529Q) derived from the Human Factor H interaction site. Co-expression of FH9 improved the stability of FP1 by preventing a proteolytic cleavage within the PspCN peptide.

FH9 had the sequence:
KSCDIPVFMNARTKNDFTWFKLQDTLDYECHDGYESNTGSTTGSIVCGYNGWSDLPICYER (SEQ ID NO:4)

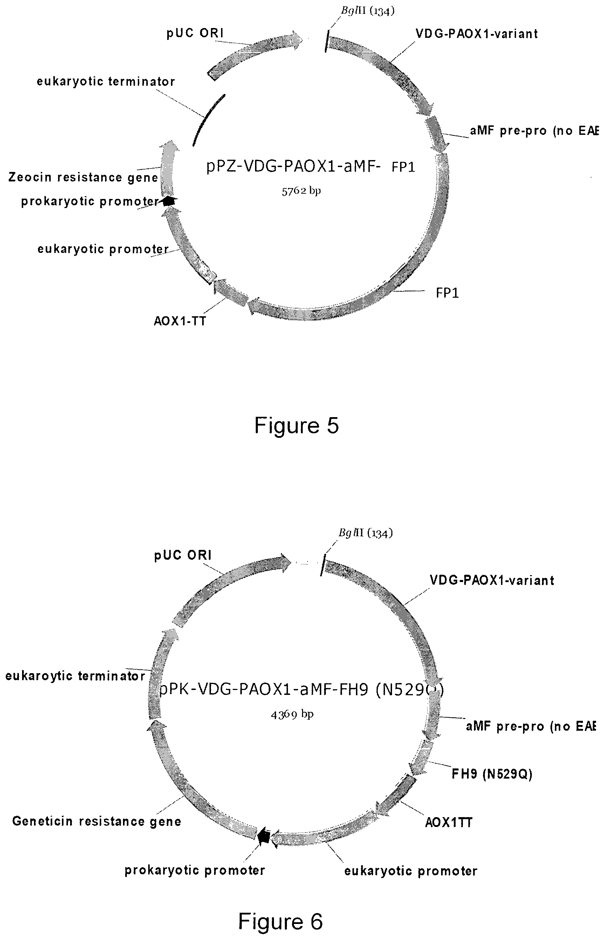

Expression plasmids for FP1 and FH9 are shown in Figures 5 and 6 respectively.

The nucleotide sequence (SEQ ID NO:5) of the expression unit VDG-PAOX1-variant-aMF-FP1 confirmed by sequencing in double strand coverage with individual sequence features identified below. 
atcattattagcttactttcataattgcgactggttccaattgacaagcttttgattttaacgacttttaacgacaacttgagaagatcaa
aaaacaactaattattcgaaGAATTCCGAAACGATGAGATTCCCATCTATTTTCACCGCTGTCTTG
 
TTCGCTGCCTCCTCTGCATTGGCTGCCCCTGTTAACACTACCACTGAAGACGAGACTGC
TCAAATTCCAGCTGAAGCAGTTATCGGTTACTCTGACCTTGAGGGTGATTTCGACGTCG
CTGTTTTGCCTTTCTCTAACTCCACTAACAACGGTTTGTTGTTCATTAACACCACTATCG
CTTCCATTGCTGCTAAGGAAGAGGGTGTCTCTCTCGAGAAGAGAGATGCACACAAATCA
GAAGTTGCTCATAGATTCAAGGACCTCGGAGAAGAGAACTTCAAGGCTCTTGTCCTTAT
CGCTTTCGCTCAATACCTTCAGCAATGTCCTTTTGAGGACCACGTTAAGTTGGTGAACG
AAGTTACCGAGTTCGCTAAAACTTGCGTAGCTGACGAATCTGCTGAGAACTGTGACAAG
TCACTTCACACTCTTTTTGGTGACAAGCTTTGTACTGTCGCTACCCTTCGTGAAACCTAC
GGCGAAATGGCCGATTGCTGTGCTAAGCAGGAACCTGAAAGAAACGAATGTTTCTTGC
AGCACAAGGACGATAACCCCAATCTTCCTCGTTTGGTTCGTCCTGAGGTCGACGTTATG
TGCACCGCTTTTCATGACAACGAAGAGACTTTCTTAAAGAAATACCTTTACGAAATCGCT
CGTCGTCACCCATACTTCTACGCTCCAGAGCTGTTGTTCTTCGCAAAGAGATATAAGGC
TGCTTTCACTGAGTGTTGCCAAGCTGCTGACAAGGCAGCTTGTCTATTGCCTAAGCTTG
ACGAATTGCGAGATGAGGGTAAAGCATCTTCCGCCAAGCAGAGATTGAAATGCGCTTC
CTTGCAGAAGTTTGGTGAGCGAGCTTTCAAAGCCTGGGCCGTGGCTAGGTTGAGCCAA
CGTTTTCCTAAAGCTGAGTTCGCTGAAGTTTCTAAGTTGGTTACTGATCTTACTAAGGTG
CACACTGAATGTTGCCACGGTGACCTTCTGGAGTGTGCTGATGACCGTGCAGATTTGG
CTAAGTATATTTGTGAAAACCAAGATTCTATTTCTTCTAAACTAAAGGAATGTTGTGAAAA
GCCACTTCTTGAGAAAAGTCACTGTATCGCTGAGGTGGAGAACGACGAGATGCCAGCT
GACCTTCCTAGCCTGGCTGCTGATTTCGTTGAATCTAAGGACGTATGCAAGAATTACGC
AGAGGCCAAGGATGTTTTCCTTGGCATGTTTTTGTACGAGTACGCTAGAAGACACCCTG
ACTACTCCGTAGTTCTCTTGCTGAGGTTGGCAAAGACCTACGAGACTACCCTAGAGAAG
TGTTGCGCCGCAGCTGATCCTCACGAGTGTTATGCTAAAGTTTTTGATGAGTTTAAACC
TTTGGTTGAGGAGCCACAAAACTTGATTAAGCAGAACTGCGAGCTTTTCGAACAATTGG
GGGAATACAAGTTCCAAAATGCCTTGCTAGTCAGGTACACCAAAAAGGTCCCTCAGGTC
AGCACCCCAACCTTAGTCGAGGTGTCCAGAAATTTGGGCAAAGTTGGTTCTAAATGTTG
CAAGCACCCAGAAGCTAAGAGGATGCCATGTGCCGAAGACTACCTTTCCGTCGTTCTG
AACCAACTCTGTGTTTTGCACGAAAAGACTCCAGTCTCAGACCGTGTCACGAAATGTTG
TACCGAGTCTCTGGTTAACAGAAGACCTTGTTTCTCTGCTTTGGAAGTTGACGAAACTTA
CGTCCCAAAGGAGTTCAACGCGGAGACTTTCACCTTCCACGCCGACATTTGTACACTTT
CCGAGAAGGAAAGACAAATCAAGAAGCAAACCGCACTAGTTGAATTGGTTAAACATAAG
CCTAAGGCTACCAAAGAACAATTGAAAGCAGTTATGGATGATTTTGCGGCTTTCGTGGA
AAAGTGTTGTAAGGCTGATGACAAGGAAACCTGTTTCGCCGAAGAAGGTAAGAAGTTAG
TCGCCGCCTCTCAGGCTGCTCTTGGACTGTCCGAGAGAGACAAGGCTAGAAAGGAAGT
TGAAGAGTATGTGAAAAAGATTGTTGGTGAGTCTTACGCTAAGTCTACTAAAAAGCGTC
ACACTATCACCGTCGCCCTTGTTAACGAGTTGAATAACATTAAAAACGAATACCTCAACA
AGATCGTCGAATCTACCTCTGAATCTCAGCTACAAATTCTGATGATGGAATCCAGATCC
 
AAGGTTGATGAGGCAGTATCAAAGTTCGAGAAGTAATAGCGGCCGCTCAAGAGGATGT
CAGAATGCCATTTGCCTGAGAGATGCAGGCTTCATTTTTGATACTTTTTTATTTGTAACC
TATATAGTATAGGATTTTTTTTGTCATTTTGTTTCTTCTCGTACGAGCTTGCTCCTGATCA
GCCTATCTCGCAGCAGATGAATATCTTGTGGTAGGGGTTTGGGAAAATCATTCGAGTTT
GATGTTTTTCTTGGTATTTCCCACTCCTCTTCAGAGTACAGAAGATTAAGTGAGACCTTC
GTTTGTGCGGATCCTTCAGTAAtgtct
 VDG-AOX1-promoter variant identified (lower case), Cloning site and Kozak sequence (capitals), Alpha-mating factor (aMF) pre-pro (without EAEA) from *S*. *cerevisiae* (capitals underlined), Gene sequence FP1 (capitals), Double stop codon and cloning site (capitals bold), AOX1 transcription terminator (capitals underlined), Start of eukaryotic promoter for Zeocin-gene (capitals).

The nucleotide sequence (SEQ ID NO:6) of the expression unit VDG-PAOX1-variant-aMF-FH9-(N529Q) confirmed by sequencing in double strand coverage with individual sequence features identified below.

VDG-AOX1-promoter variant (capitals), Cloning site and Kozak sequence (capitals in bold), Alpha-mating factor pre-pro (without EAEA) from *S*. *cerevisiae* (capitals), Gene sequence FH9-(N529Q) (capitals underlined), Double stop codon and cloning site (capitals), AOX1 transcription terminator (capitals bold), Eukaryotic with integrated prokaryotic promoter for KanMX-gene (capitals).

### Start of KanMX-gene

ATCATTATTAGCTTACTTTCATAATTGCGACTGGTTCCAATTGACAAGCTTTTGATTTTAA
CGACTTTTAACGACAACTTGAGAAGATCAAAAAACAACTAATTATTCGAAGAATTCCGAA
ACGATGAGATTCCCATCTATTTTCACCGCTGTCTTGTTCGCTGCCTCCTCTGCATTGGC
TGCCCCTGTTAACACTACCACTGAAGACGAGACTGCTCAAATTCCAGCTGAAGCAGTTA
TCGGTTACTCTGACCTTGAGGGTGATTTCGACGTCGCTGTTTTGCCTTTCTCTAACTCC
ACTAACAACGGTTTGTTGTTCATTAACACCACTATCGCTTCCATTGCTGCTAAGGAAGAG
GGTGTCTCTCTCGAGAAGAGAAAGTCCTGCGACATCCCTGTTTTCATGAATGCTAGAAC
TAAAAACGATTTCACTTGGTTTAAGCTTCAAGACACTCTCGATTACGAGTGTCACGACG
GTTACGAATCTAACACCGGATCAACCACAGGTTCTATTGTCTGTGGTTACAACGGTTGG
TCTGACCTGCCAATTTGTTATGAGAGATAATAGCGGCCGCTCAAGAGGATGTCAGAAT
GCCATTTGCCTGAGAGATGCAGGCTTCATTTTTGATACTTTTTTATTTGTAACCTATATA
GTATAGGATTTTTTTTGTCATTTTGTTTCTTCTCGTACGAGCTTGCTCCTGATCAGCCTA

TCTCGCAGCAGATGAATATCTTGTGGTAGGGGTTTGGGAAAATCATTCGAGTTTGATG
TTTTTCTTGGTATTTCCCACTCCTCTTCAGAGTACAGAAGATTAAGTGAGACCTTCGTT
TGTGCGGATCCTTCAGTAATGTCTTGTTTCTTTTGTTGCAGTGGTGAGCCATTTTGACTT
CGTGAAAGTTTCTTTAGAATAGTTGTTTCCAGAGGCCAAACATTCCACCCGTAGTAAAGT
GCAAGCGTAGGAAGACCAAGACTGGCATAAATCAGGTATAAGTGTCGAGCACTGGCAG
GTGATCTTCTGAAAGTTTCTACTAGCAGATAAGATCCAGTAGTCATGCATATGGCAACAA
TGTACCGTGTGGATCTAAGAACGCGTCCTACTAACCTTCGCATTCGTTGGTCCAGTTTG
TTGTTATCGATCAACGTGACAAGGTTGTCGATTCCGCGTAAGCATGCATACCCAAGGAC
GCCTGTTGCAATTCCAAGTGAGCCAGTTCCAACAATCTTTGTAATATTAGAGCACTTCAT
TGTGTTGCGCTTGAAAGTAAAATGCGAACAAATTAAGAGATAATCTCGAAACCGCGACT
TCAAACGCCAATATGATGTGCGGCACACAATAAGCGTTCATATCCGCTGGGTGACTTTC
TCGCTTTAAAAAATTATCCGAAAAAATTTTCTAGAGTGTTGACACTTTATACTTCCGGCTC
GGATAATACGACAAGGTGTAAGGAGGACTAAACCATGGGTAAGGAAAAGACTCACGT

The FP1 and FH9 expression plasmids were used to transform *Komagatella phaffii* cells, expression clones selected on the basis of antibiotic resistance and characterized for optimized expression in culture supernatants.

### Priming of Dialysis Filters using FP1 Medical Device.

FP1 is designed to coat extracorporeal circuits, in particular, those used for haemodialysis and a number of *ex vivo* studies have demonstrated the ability of FP1 to coat dialysis filters and maintain biological activity (Factor H binding and complement inhibition).

Preliminary studies demonstrated that FP1 was unaffected by the dialysis fluid as the SE-HPLC profile and K_{D} were identical to FP1 stock in DPBS (Table 1 below).

**Table 1. FP1 K_{D} in DPBS and Fresenius dialysis fluid. Results from two independent K_{D} determinations for FP1 in DPBS and dialysis fluid are shown**

| | **K_{D} (nM)** | **K_{D} (nM)** |
|---|---|---|
| FP1 Ref DPBS | 0.85 | 0.97 |
| FP1 DF1+34 | 0.75 | 0.94 |

Dialysis fluid (Fresenius A/453 acetate/bicarbonate 1+34 dialysis fluid) was equilibrated to 37°C in the water bath and 1 litre pumped through the connected dialysis filter at 40ml/min. After flushing, FP1 was injected into the circuit via the 3-way valve, 5mg (5ml at 1mg/ml) are injected over 30 secs. The outlet fluid from the dialyser is collected in 50ml aliquots (10x 50ml fractions) and the filter washed for 4hrs by circulating 4x 100ml dialysis fluid (100ml each hour was circulated) to determine if any FP1 eluted from the filter. FP1 quantitation was performed to determine the unbound fraction and any FP1 in the 4x100ml circulated fractions. The difference between the load (5mg) and the FP1 recovered in the unbound and leached fractions is equivalent to the FP1 coating the filter.

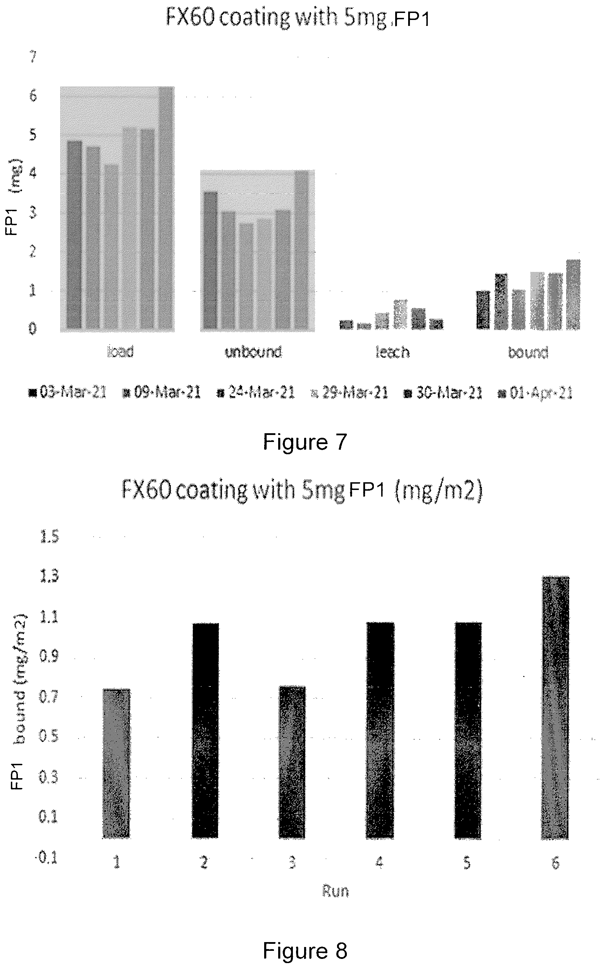

Results from six independent priming experiments using Fresenius FX60 dialysis filters are shown in Figures 7 and 8. FP1 quantitation was performed on the load, unbound and wash fractions and the load determined by subtraction of unbound + wash from the load (Table 2).

**Table 2. Summary of 6x Fresenius FX60 priming experiments.**

| | **mg FP1** | | | | **FP1 mg/m²** |
|---|---|---|---|---|---|
| | **load** | **unbound** | **wash** | **Bound** | |
| Ave | 5.09 | 3.25 | 0.43 | 1.41 | 1.01 |
| +/- SD | 0.66 | 0.51 | 0.23 | 0.31 | 0.22 |

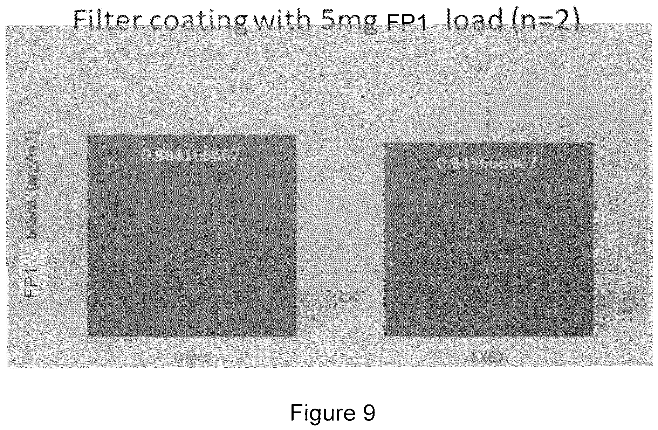

In a separate experiment, the loading for 2x FX60 and 2x Nipro (Elisio-15H) dialysis filters was determined and shown to be similar (Fig 9).

In some instances, filters were stained with FP1-specific antibodies and filter-bound FP1 observed using secondary antibodies/HRP conjugates and DAB colour development.

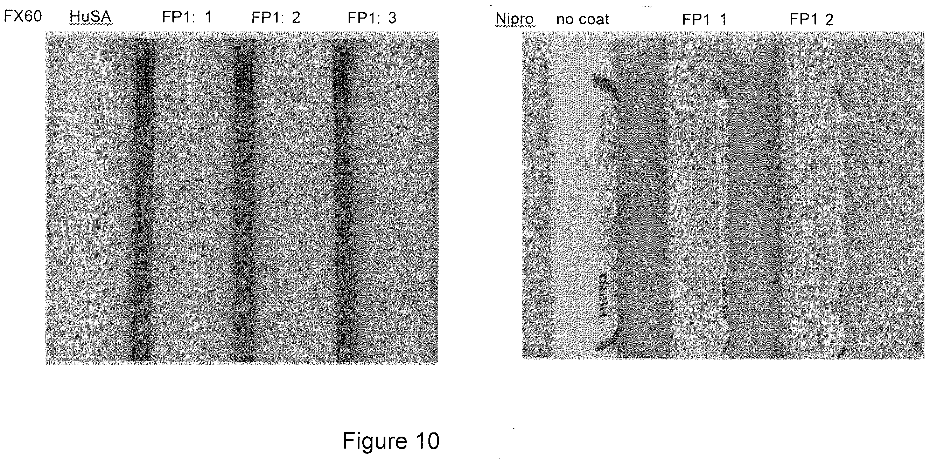

Results for staining of 3 FX60 and 2 Nipro Elisio-15H filters from the above experiments are shown in the Figure 10 and indicate that the filters retain FP1 after priming, flushing and extensive washing.

### Testing of FP1 modified dialysis

In preparation for the First in Human study, priming was tested using a Fresenius 6008 dialysis machine. Fresenius FX100 dialysis filters were used but quantitation of the unbound FP1 after priming was not possible using this machine as all the flush and wash material could not be collected. Small volume sampling was possible at two ports in the dialysis machine and 1ml samples were collected every minute for 15mins after priming.

Protocol for 6008 priming:-
- 6008 equilibration of tubing set and FX100 (2.2m²)
   - c1litre dialysis fluid/variable flow rate
- Pre-circulation mode (waiting for patient)
   - H Guard injected into system vial LMW Heparin line
   - 5ml at 1mg/ml over 30 secs
   - Flush with 5ml DPBS
- Single pass through filter/tubing set
   - Samples collected at two ports at 1 minute intervals for 15 mins
   - Port A (n=4)
   - Port A and B (n=2)
- FP1 quantitation in samples/staining of filters with K45

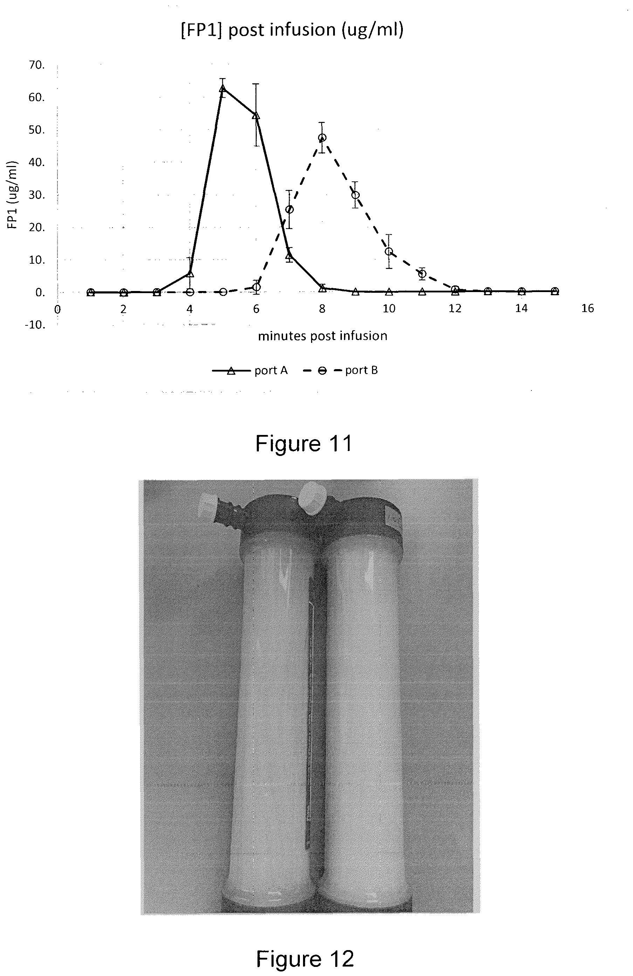

FP1 quantitation confirmed priming of the filters with unbound FP1 detected in samples during the first 8 minutes (Fig 11) and subsequent FP1 antigen staining confirmed loading of the filters during priming.

FP1 priming using 4x FX800 Cordiax filters to be used in the First in Human study was also tested using an ex vivo circuit and the load determined from the unbound fractions. Results are presented in the Table 3 below, the dialysis surface area for a Fresenius FX800 Cordiax filter is 2m² resulting in an A0T load of ~ 1mg/m². This coating density is similar to the results for the FX60 and Nipro Elisio-15H presented above.

**Table 3. FP1 load for 4xFX800 Cordiax filters primed with 5mg FP1.**

| **Run** | **FP1 coat (mg)** | **Load** / **m²** |
|---|---|---|
| 1 | 1.77 | 0.885 |
| 2 | 2.38 | 1.19 |
| 3 | 1.64 | 0.82 |
| 4 | 1.9 | 0.95 |
| average | 1.92 | 0.96 |

### FP1 priming of AN69ST filters used in CRRT

Different dialysis filters are used for continuous renal replacement therapy (CRRT) and a single filter can be used continuously for 24-72hrs circulation. A typical CRRT filter is the Nephral 400 using AN69ST hollow fibres. AN69ST membranes offer excellent biocompatibility as the Polyethylenimine (PEI) surface treatment neutralizes the membrane's surface charge without reducing the bulk adsorption of low- and medium-sized molecules.

Using an ex vivo priming and circulation system, the priming and properties of AN69ST filters were investigated.

Priming, flushing and washing of AN69ST filters was performed as described previously with the following exceptions:-
- In some instances, circulation was maintained for 24hrs
- During circulation, in some instances, 50% human serum was circulated for 24hrs in place of dialysis fluid

Antigen staining using the anti-FP1 polyclonal K45 and the anti-human FH monoclonal Ox24 was also performed on some filters.

FP1 binding capacity of AN69ST filters.
- H Guard priming:- 5ml at 1mg/ml injected over 30 secs via sample/injection port.
- 6x 50ml dialysis effluent collected during priming.
- Additional 350ml/filter collected in bulk
- FP1 Quantitation using Octet method
   > FP1 bound to filter = load - unbound FP1 in fractions.

12 AN69ST filters were primed with 5mg FP1 (5ml at 1mg/ml) and the unbound FP1 in the washout fractions used to determine the filter loading. Results are presented in Table 8 below and, as the dialysis area for Nephral 400/AN69ST is 1.65m², this was used to determine the average load/m² for AN69ST filters.

The average FP1 loading capacity for the AN69ST filters (1.3mg/m²) is greater than the loading capacity for the Fresenius FX series (1mg/m²) determined previously and this is probably due to the different compositions of the two filter types.

**Table 4. Average FP1 load for 12x Nephral 400 AN69ST filters primed with 5mg FP1.**

| | **Load (mg)** | **Ave bound (mg)** | **+/- SD** | **Ave load mg/m²** |
|---|---|---|---|---|
| AN69ST (n=12) | 5 | 2.15 | 0.302 | 1.30 |

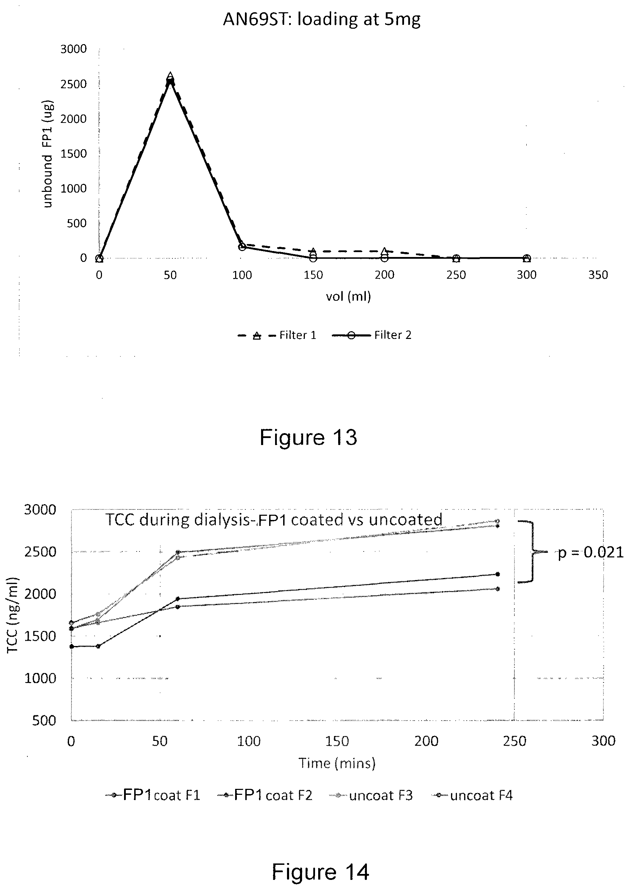

FP1 quantitation of samples collected during priming of 2xAN69ST filters indicates that the majority of the excess FP1 is flushed from the system in the first 50 ml with only traces amounts of FP1 detectable in the remaining fractions (Figure 13)

### FP1 coating inhibits complement activation on AN69ST filters.

After priming with 5mg FP1, 50% human serum was circulated around 2x FP1 primed and 2x uncoated control AN69ST filters for 24hrs and samples removed for analysis as various time points. Complement components C5b-9 (TCC), C5a and C3a were measured using commercially available ELISA kits in the time course samples and the results presented in Figure 14-16.

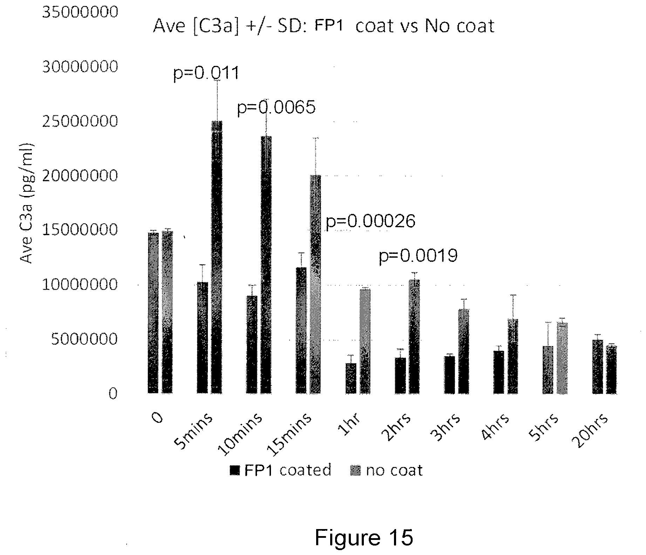

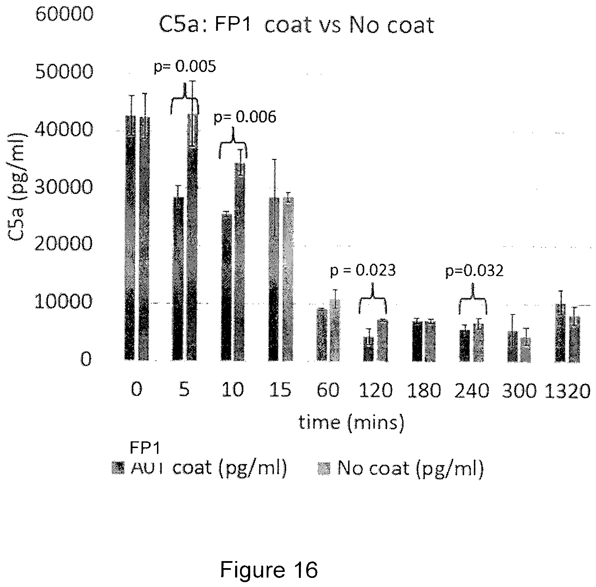

Significant reductions in C5b-9, C5a and C3a were observed with the FP1-primed AN69ST filters (Figures 14, Tables 5 and 6). The inhibition of complement activation with the FP1-coated filters was most noticeable for C3a and C5a at early times of dialysis circulation (see Figs 15 and 16 and Tables 5 and 6) as, due to their small molecular size (~10kDa), they will be dialysed out of the circulation (note the large drop in concentrations after 1hr in Figs 15 and 16) whereas, by virtue of the high molecular size of C5b-9 (1.2MDa), it will be retained in the dialysis circuit.

**Table 5. Average C3a concentration for duplicate samples (assayed in duplicate, n=4) for 2xFP1 coated vs uncoated AN69ST filters.**

| | FP1 coat | no coat | FP1 coated | no coat | |
|---|---|---|---|---|---|
| time | ave C3a (pg/ml) | | +/- SD | | T Test FP1 coat vs No coat |
| 0 | 14,784,673.2 0 | 14,941,593.6 0 | 196,074.51 | 211,311.41 | 0.090 |
| 5mins | 10,311,133.2 7 | 25,081,348.9 7 | 1,539,594.9 5 | 3,695,894.9 5 | 0.011 |
| 10mins | 9,031,752.97 | 23,690,780.5 8 | 970,964.46 | 3,356,764.4 4 | 0.007 |
| 15mins | 11,606,526.8 4 | 20,082,635.1 4 | 1,351,308.6 6 | 3,413,247.4 0 | 0.037 |
| 1hr | 2,850,772.31 | 9,656,059.69 | 724,192.72 | 150,302.60 | 0.000 |
| 2hrs | 3,363,404.10 | 10,529,403.5 1 | 768,265.05 | 627,077.65 | 0.002 |
| 3hrs | 3,509,726.81 | 7,828,344.32 | 183,606.51 | 896,940.45 | 0.001 |
| 4hrs | 4,013,036.41 | 6,945,969.06 | 442,345.81 | 2,179,266.2 6 | 0.098 |
| 5hrs | 4,452,447.52 | 6,623,766.83 | 2,170,537.5 6 | 389,953.24 | 0.188 |
| 20hrs | 5,057,340.69 | 4,501,218.34 | 450,811.28 | 166,752.55 | 0.150 |

**Table 6. Average C5a concentration for duplicate samples (assayed in duplicate, n=4) for 2xFP1 coated vs uncoated AN69ST filters.**

| time | FP1 coat, C5a (pg/ml) | No coat, C5a (pg/ml) | FP1 +/- SD | No coat +/- SD | T Test FP1 coat vs No coat |
|---|---|---|---|---|---|
| 0 | 42,684.09 | 42,478.46 | 3,464.82 | 3,906.94 | 0.545 |
| 5mins | 28,576.30 | 43,019.61 | 1,857.64 | 5,761.53 | 0.005 |
| 10mins | 25,537.25 | 34,504.06 | 407.07 | 2,213.33 | 0.006 |
| 15mins | 28,422.33 | 28,516.58 | 6,656.29 | 869.36 | 0.976 |
| 1hr | 9,062.75 | 10,784.25 | 235.03 | 1,543.95 | 0.145 |
| 2hrs | 4,227.90 | 7,196.10 | 1,385.77 | 127.44 | 0.023 |
| 3hrs | 7,053.89 | 6,988.13 | 405.74 | 318.17 | 0.790 |
| 4hrs | 5,619.69 | 6,651.17 | 779.80 | 962.83 | 0.032 |
| 5hrs | 5,429.08 | 4,365.91 | 2,847.63 | 1,493.18 | 0.618 |
| 20hrs | 10,318.33 | 8,056.64 | 2,078.74 | 1,576.07 | 0.073 |

FP1 and FH antigen staining was performed after 50% human serum was circulated around 4x AN69ST filters primed with 5mg FP1. After priming, 500ml 50% human serum/filter was circulated for 4hrs. Each filter was then washed with 1 litre DPBS and FP1 or FH antigen staining of filters performed using antibodies specific for FP1 (K45, n=2) and Factor H (Ox24, n=2). Antigen staining was performed over 20hrs and then each filter was washed with 1.25 litres DPBS.

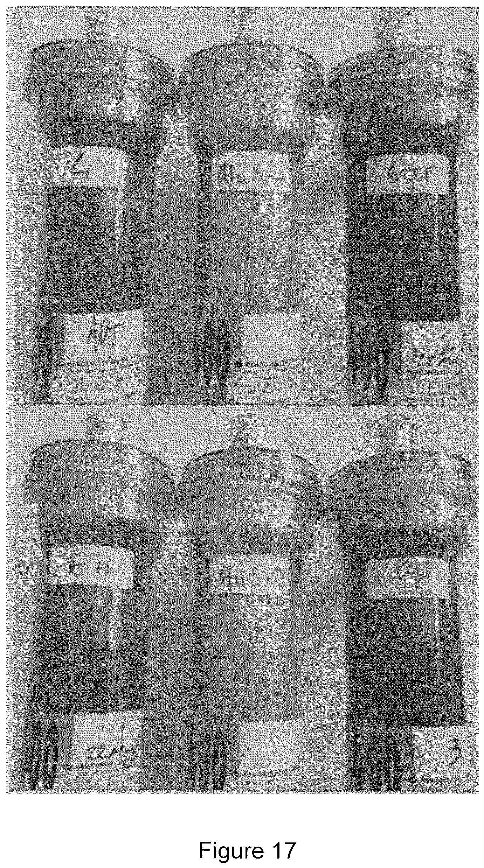

Second antibody/HRP conjugates (goat anti-Rb IgG/HRP conjugate for K45 and goat antimouse IgG/HRP conjugate for Ox24) was circulated for 4hrs followed by a further wash with 1 25litres DPBS/filter. DAB substrate was then circulated and heavy DAB staining of filters for both FP1 and FH was observed (Figure 17) with minimal background staining with a control HuSA-primed AN69ST filter.

Thus, FP1 priming and Human serum circulation demonstrates that filter bound FP1 captures FH from the circulation and the complex acts to inhibit complement activation.

### Hemocompatibility of FP1 Modified FX80 Dialyzers in Human Blood ex-vivo

An *ex vivo* haemocompatibility study using whole human blood was performed.

### Methods

Freshly donated human blood (Medical Service, ICO Obernburg, Germany) was used within 1 h after blood drawing. Blood was anticoagulated with 3.5 IU/ml unfractionated heparin (#H3149, Sigma Aldrich, Steinheim, Germany). The following blood donors were chosen for the experiments (compare with the donor numbers in report eXcorLab 0122):
1st experiment: No. 2: Blood group: B positive, blood volume: 500 mL
2nd experiment: No. 25: Blood group: A positive, blood volume: 500 mL
3rd experiment: No. 24: Blood group: 0 positive, blood volume: 500 mL

Blood from one donor was divided and used for both filter types (250 mL per filter No. 1-2). This setup was repeated three times per filter type, leading to three independent measurements with three different blood donors (n=3, respectively), in which the coated and non-coated filters were compared with the same donor blood. The reference filter (a non-coated FX80 dialyzer) was primed by recirculating 1 L of saline through the blood compartment using a flow rate of 250 mL/min for 30 min. The test filter was primed and coated by recirculating 1 L of FP1 working solution (5 mg/L FP1 in saline, 250 mL/min) for 30 min.

A key finding from this ex vivo hemocompatibility study related to the complement components C5a and soluble C5b-9. The physiological reference range for C5a is from 0.15 to 0.5 ng/mL and for sC5b-9 is from 75-219 ng/mL. Higher C5a and sC5b-9 concentration means activation of the complement system. Therefore, slight complement activation was detected for both dialyzer types, in which the test filter after FP1 coating showed statistically significantly lower sC5b-9 and C5a values after 240 min as compared to the non-coated reference filter. Therefore, the FP1 coating seems to have a positive effect on reducing complement activation.

### Clinical Studies

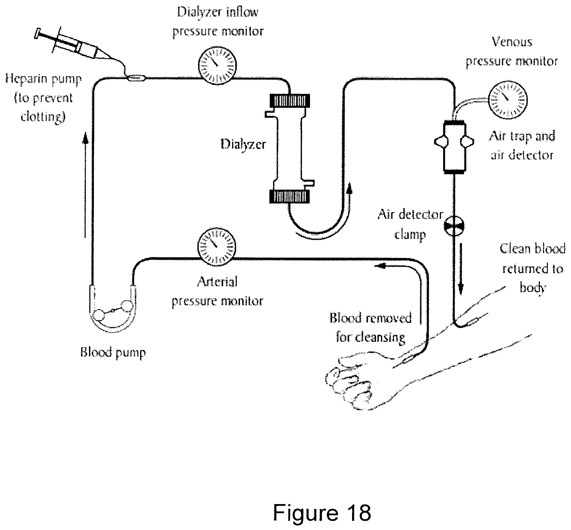

Preclinical testing and dialysis filter performance studies, FP1 was tested in a First in Human clinical study. The Study tested FP1 as a Medical Device for priming tubing sets and dialyser filters in patients undergoing haemodialysis for chronic kidney disease. A typical set up for these studies is shown in Figure 18.

### Description of Overall Study Design

This prospective, open-label, study was conducted in accordance with the requirements of EN ISO 14155, the Declaration of Helsinki (revised version of Edinburgh, Scotland 2000), Good Manufacturing Practice (GMP), Good Clinical Practice (GCP) and the current national regulations and guidelines, approved by both the local ethics committee and regulatory authority.

The study was performed in a stable patient population who are on haemodialysis and who have a blood biomarker profile at screening, suggesting an increased risk of sensitivity to the haemodialysis dialyser and/or blood tubing sets (C3 deposition assay ratio <0.3 - measured immunologically using a C3 antibody in FP1 vs human serum albumin coated ELISA plates). Participants were recruited based on the screening safety assays and attended a total of five-six consecutive visits during the clinical trial
1. Screening [up to 30 days prior to the start of the clinical trial] (Visit #1)
2. A non-interventional haemodialysis using standard priming solutions [Baseline - mid-week session: Day 0] (Visit #2)
3. For WoCBP - a serum hCG test was repeated between days 1-6 prior to intervention (Visit #3)
4. A single haemodialysis using FP1 to first prime the dialyser and tubing set [midweek session: Day 7] (Visit #4)
5. Followed by a further non-interventional haemodialysis without FP1 [i.e. using standard priming solutions] [first haemodialysis post intervention] (Visit #5)
6. Finally a follow up visit 14-21 days post intervention to perform antibody analysis. (Visit #6)

For the purposes of safety, 1 sentinel participant was recruited and followed for safety through to the end of Visit #4 before general recruitment to the remainder of the cohort were opened. However, screening activities did not pause.

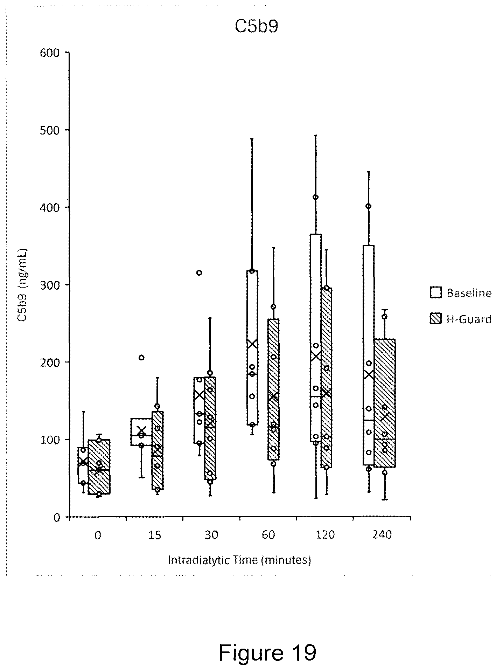

The study shows that complement is activated during dialysis and that the use of FP1 ("H-Guard") significantly impacts the measurable levels of C5b9 (Figure 19), especially after intradialytic times of 60 minutes or more.

### Results from First in Human FP1 intervention in Chronic Dialysis Patients.

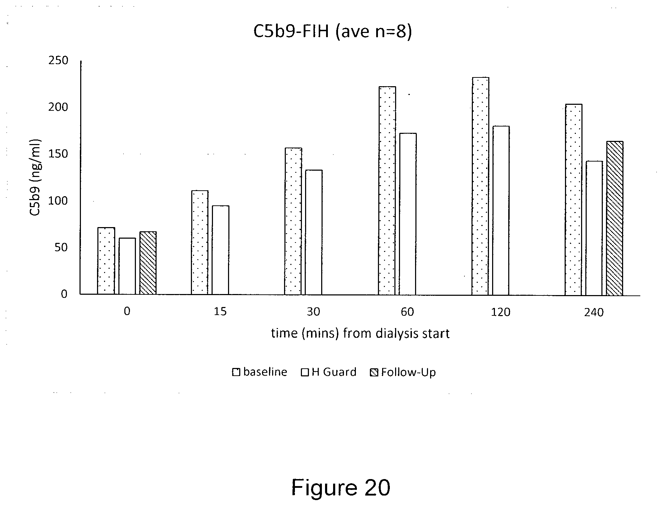

The FIH study was designed to determine the effects of FP1 during dialysis. One week after a standard dialysis session (baseline) the participants (n=8) were dialysed using an FP1-primed filter and a further standard dialysis session followed 48hrs later. Plasma samples were collected from each participant during the baseline, FP1 ("H Guard") and follow-up session as indicated in the Figure 20.

C5b9 is the end product of complement activation (aka Terminal Complement Complex (TCC) or Membrane Attack Complex (MAC)) and its concentration was determined in the intradialytic samples collected from each patient.

The appearance of C5b9 indicates that the complement cascade has been activated and the additional pro-inflammatory factors, such as the anaphylatoxins C3a and C5a, have been released. Thus, increased C5b9 during dialysis is indicative of complement activation and the consequential complement-driven inflammation.

Complement is activated during dialysis in the 8 study participants with peak activity observed between 1-2 hours (Figure 20). Comparison of C5b9 levels during the FP1 intervention and baseline sessions demonstrated a reduction in complement activation during the FP1 session (Figures 19 and 20).

Surprisingly, the reductions of C5b9 levels observed during the FP1 session were also present in the follow-up session. Therefore, FP1 not only reduced complement activation during the primed session but also demonstrated a systemic effect during the follow-up session (Figure 20).

While there has been hereinbefore described approved embodiments of the present invention, it will be readily apparent that many and various changes and modifications in form, design, structure and arrangement of parts may be made for other embodiments without departing from the invention and it will be understood that all such changes and modifications are contemplated as embodiments as a part of the present invention as defined in the appended claims.

## Claims

1. A fusion protein having a structure A-L-B, wherein:
A comprises a sequence that has at least 90% similarity to SEQ ID NO :1 ;
L comprises a linker or a chemical bond;
B comprises a sequence that has at least 90% similarity to SEQ ID NO:2.

2. The fusion protein of claim 1, wherein L is a chemical bond.

3. The fusion protein of claim 1 or claim 2, wherein A comprises SEQ ID NO:1.

4. The fusion protein of any preceding claim, wherein B comprises SEQ ID NO:2.

5. The fusion protein of any preceding claim, wherein the A is SEQ ID NO:1, L is a chemical bond, and B is SEQ ID NO:2.

6. A fusion protein comprising SEQ ID NO:3.

7. A medical device, wherein the surface of the medical device is at least partially coated with the fusion protein of any one of claim 1 to claim 6.

8. The medical device of claim 7, wherein substantially all of the external surfaces of the device (i.e. those which will be in contact with body tissues and/or fluids) are coated with the fusion protein.

9. The medical device of any one of claim 7 or claim 8, wherein the device is coated with a composite of the fusion protein of any one of claim 1 to claim 6 bound to CFH.

10. The medical device of any one of claim 7 to claim 9, wherein the medical device is an implantable medical device or a microcapsule.

11. The medical device of any one of claim 7 to claim 10, wherein the device is a filter medium.

12. The medical device of any one of claim 7 to claim 11, wherein the device is configured to be used in haemodialysis.

13. The medical device of any one of claim 7 to claim 12, wherein the device is configured to be incorporated into an extracorporeal circuit.

14. The fusion protein of any of claim 1 to claim 6 for use in the treatment or prevent of a disease, condition or other medical complication associated with aberrant complement regulatory activity in a subject or where reducing complement activity is beneficial, optionally wherein the disease of condition or other medical complication is a consequence of inflammation or induces inflammation.

15. The fusion protein for the use of claim 14, wherein the disease, condition or other medical condition is one or more of:
- Sepsis;
- Traumatic brain injury;
- Ischaemic Reperfusion injury;
- Transplant associated Thrombotic Microangiopathies
- complement mediated neurological damage.
